# EUROPEAN PATENT SPECIFICATION

(11) **EP 2 548 448 B1**
(45) Date of publication and mention of the grant of the patent: **10.05.2017**
(21) Application number: 12174415.5
(22) Date of filing: 29.06.2012
(51) Int. Cl.: A61Q 1/02, A61Q 1/12, A61Q 5/00, A61Q 5/02, A61Q 5/06, A61Q 5/12, A61Q 17/04, A61Q 19/00, A61Q 19/08, A61Q 19/10, A61K 8/02, A61K 8/97, A61K 36/899, A61Q 1/06, A23P 30/20, A23L 29/225, A23L 7/10

(54) **Novel cereal products, production and use thereof, and cosmetic formulations containing them**
Neuartige Getreideprodukte, Herstellung und Verwendung davon und kosmetische Formulierungen damit
Nouveaux produits céréaliers, la production et l'utilisation de ceux-ci et des formulations cosmétiques les contenant

(30) Priority: 18.07.2011 FI 20115758; 18.07.2011 US 201161508706 P
(43) Date of publication of application: 23.01.2013
(73) Proprietor: OAT Services Ltd, Southampton SO16 7FU (GB); Polar Glucan Oy, 00990 Helsinki (FI)
(72) Inventor: MAUNSELL, Cark, Southampton, SO16 7FU (GB); MYLLYMÄKI, Olavi, 00990 Helsinki (FI); HUHTAKALLIO, Martti, 61600 Jalasjärvi (FI); LEHTOMÄKI, Ilkka, 00990 Helsinki (FI)
(74) Representative: Seppo Laine Oy

(56) References cited:
- WO-A1-2005/122778
- WO-A1-2011/065880
- WO-A2-2010/140963
- US-A1- 2007 184 186
- GUTKOSKI L. C., EL-DASH A. A.: "Effect of extrusion process variables on physical and chemical properties of extruded oat products", PLANT FOODS FOR HUMAN NUTRITION, vol. 54, 1999, pages 315-325, XP002685505,
- GUALBERTO D. G., BERGMAN C. J., KAZEMZADEH M.: "Effect of extrusion processing on the soluble and insoluble fiber and phytic acid contents of cereal brans", PLANT FOODS FOR HUMAN NUTRITION, vol. 51, 1997, pages 187-198, XP002685506,
- LIU Y., HSIEH F., HEYMANN H., HUFF H.E.: "Effect of Process Conditions on the Physical and Sensory Properties of Extruded Oat-Corn Puff", JOURNAL OF FOOD SCIENCE, vol. 65, no. 7, 2000, XP002685507,
- "Quadrumat Senior", , 10 October 2016 (2016-10-10), Retrieved from the Internet: URL:http://www.brabender.com/english/food/ products/sample-preparation/grinding/quadr umatr-senior.html
- "Gorgens TurboRotor System", 10 October 2016 (2016-10-10)
- "Particle Size Conversion Table", 10 October 2016 (2016-10-10)
- "USP monographs Colloidal Oatmeal", 10 October 2016 (2016-10-10)
- Lachman, Lieberman, Kanig: "The Theory and Practise of Industrial Pharmacy", 1976 pages 470-474,
- "USP Monograph USP32- NF27", UNITED STATES PHARMACOPEIA. NATIONAL FORMULARY. SUPPLEMENT, UNITED STATES PHARMACOPEIAL CONVENTION, ROCKVILLE, MD, US, 1 January 2010 (2010-01-01), page 2024, XP009192285, ISSN: 0190-5384 ISBN: 978-1-889788-13-5
- ELLEN S KURTZ, WARREN WALLO: "Colloidal Oatmeal: History, Chemistry and Clinical Properties", JOURNAL OF DRUGS IN DERMATOLOGY, 2007,

## Description

### Field of the Invention

This invention relates to novel oat cereal products having enhanced colloidal properties. Particularly this invention relates to oat products that are suitable for use in cosmetic and food applications. Further this invention relates to a method of producing them, their use in food and cosmetic formulations containing said cereal products.

### Description of Related Art

Cereal flours especially oat (Avena sativa) flour has been used for over a 1,000 years as an ingredient in cosmetic products to protect and repair the skin. Oat flour contains components that have anti-itching and anti-inflammatory properties. (Pomeranz 1995). Oats also contain natural antioxidants and compounds capable of absorbing UV-light (Kurtz and Wallo 2007). In Finland and the UK oats, in contrast to wheat, barley and rye, are permitted in celiac diets. Oat starch grains are similar in size (approximately 8 microns) to rice and smaller than other cereal starches. These properties make oat flour an ideal component for cosmetic products. Oat flour has been used for example in moisturising creams, anti-aging products and for use with sensitive skins for a long period. It is known that extrusion affects both physical and chemical properties of extruded oat products. Gutkoski and El-Dash (Plant Foods for Human Nutrition, 1999) studied the effects of initial moisture levels and extrusion temperatures on bulk density, water absorption and water solubility indexes, viscosity, and colour of extruded oat products. One example of an oat ingredient that is used and that has a defined specification for cosmetic industry is colloidal oatmeal (Kurtz and Wallo 2007). The colloidal oatmeal specification is described in the United States Pharmacopeial Convention USP32-NF27 page 2024, where the following characteristics are prescribed: Maximum dry matter, nitrogen, fat, protein, ash content, microbial assays, particle size and viscosity. The properties and allowable claims are described in the Federal Drug Administration (FDA) Federal Register Vol. 69, No. 160, 19th August, 2004.

Cereal flours contain a starch fraction that is easily gelatinized during heating with moisture. Gelatinisation is enhanced by mechanical energy, for example by shearing. Gelatinisation increases the viscosity of the flour in wet conditions and also modifies other properties such as the size of starch particles. It is advantageous that the level of gelatinisation is controlled for different applications, for example, cosmetics or food beverages. This invention controls the gelatinisation during extrusion.

Cereal flours may naturally have high a microbial content, which may be controlled or reduced during processing. Other typical effective control methods such as the use of chemicals or irradiation are not accepted by many cosmetic manufacturers. This invention significantly improves the microbial purity during natural extrusion and is an allowable practice by most cosmetic manufacturers.

Typical methods of controlling microbial purity are:
*Beta or Gamma Irradiation*, which produces ions that improve the microbial purity of cosmetic products. The process is limited to small finished packs or paper containers but larger unit volumes can be more problematic as the surface may become brownish and the middle of the bag may not be correctly irradiated. Irradiation can cause physicochemical changes, which are not beneficial when using the ingredient as a cosmetic product. Retail consumers may also be concerned with the use of irradiated products and the cost of this process tends to be high. The maximum allowed irradiation in the food industry is 10 kGy (kilogray) and the normal dosage in cosmetic ingredients is 2-10 kGy. Starch requires higher loads of over 5 kGy, as it is hygroscopic and may protect contaminants.

*Chemical disinfection* is an alternative method, but the chemicals may bring with them challenges such as increased allergenic activity as well as potentially altering the flour structure. These types of molecules may be prohibited by some cosmetic manufacturers.

*Hydrothermal methods* usually require high moisture and high temperature. Typical examples are pasteurisation, UHT (Ultra-high temperature treatment) and autoclaving. The parameters for these treatments may lead to significant changes in the behaviour of the flour due to gelatinisation of the starch fraction. Extrusion is also regarded as a hydrothermal method, but involves a solid material, where the moisture is low when compared to the other hydrothermal methods.

### Description of extrusion

In an extruder moistened flour is rapidly modified when it is pressurised by the force exerted by its passage along an Archimedes screw within the barrel at the end of which pressure can be further increased through the use of nozzles. An alternative to this is the use of an expander where the nozzles are replaced by hydraulic backpressure. The barrel may consist of a number of cylinders that can be either heated or cooled. The number of cylinders defines the length of barrel and the associated shaft and screws. An extruder has usually three sections described as the feeding, mixing and reaction chambers.

The extruder settings cannot be changed when the extruder is in operation. The extruder settings are for example, the different pitches and the shapes of the screws in the feeding, mixing and reaction sections, special tools in the mixing section and different nozzle settings. One of the significant settings is the use of the reverse screw, which is located in front of the nozzles to create back-pressure. Nozzles are located on a nozzle plate, and can be off different shapes and sizes. The extruder settings will usually vary according the raw material and objective of the extrusion.

The operation settings such as feed rate, speed of screw rotation, moisture and temperature can be adjusted during processing.

During extrusion normal flour moisture levels are between 15 and 35 w %. Full starch gelatinisation requires 61 w % water and 39 w % starch (Wang 1993). Therefore in traditional flour extrusion the moisture level is not sufficient for full starch gelatinisation even though the starch level of the flour is 60-70 w %. This significant phenomenon gives a means to control the degree of gelatinisation during extrusion.

*Flour characteristics:* Cereal flours consist of several components e.g. water, protein, carbohydrates (starch), fat and ash. The relative level of these components varies in different flours. These variations have a significant effect on the extrusion process. In conventional extrusions the degree of starch gelatinisation is not controlled in addition to which other components are changed. These types of extruded flours would not be the most suitable for use in cosmetics or for incorporation in certain food applications.

Oil in flour tends to create a slip layer on the surface of the starch particles during extrusion. The addition of 0.5-1.0 w % of vegetable oil decreases the mechanical energy in extrusion cooking when other extrusion variables are kept constant. The role of oils as lubricants can be observed in the low moisture extrusion of starches that have low lipid content such as potato starch, which can overheat at the contact surfaces causing problems in starch transport through the barrel and possibly blockages due to the creation of degraded material. Increasing the oil content up to 2-3% markedly prevents the starch dispersion, which decreases the melt viscosity. This significant phenomenon leads to higher levels of uncooked dough (Ilo *et al.* 2000).

Oats have higher levels of oil dispersed throughout the kernel in contrast to other cereals such as wheat, maize, rice, barley and rye, which have the majority of the oil in the germ. The germs can be easily mechanically fractioned out of the kernels during the milling process. The level of oil in oat flour is 2-3 times higher than other cereal flours (Ilo *et al.* 2000).

The extruder mechanical energy has a significant effect on the degree of gelatinisation. The level of extruder mechanical energy is affected by the following parameters:
1. The characteristics of the raw material
2. Extruder settings
   - Length of the screw
   - Screw setting
   - Nozzle setting
3. Operation settings
   - The feed rate
   - The moisture
   - The relative temperature in parts of the barrel
   - The rotational speed of the screw

The level of mechanical energy can be assessed by measuring the pressure at the entry point to the extruder nozzles. Similarly the pressure is affected by the same parameters as mechanical energy. The pressure in conventional extrusions is in the range 50-150 bar.

*Combined effect of extruder settings and flour characteristics:* It is known that the water activity is an important factor in decreasing microbial content with the use of heat. The addition of oil decreases the water activity. In an extruder the screws and especially the reverse screw create a plasticised mass, where the moist flour with added oil is very efficiently mixed whilst heated. This highly efficient mixing effectively reduces microbe count.

One drawback in the prior art solutions is that conventional extruder settings together with operational settings will create flours where the starch fraction and other flour components have been excessively modified e.g. the starch is over-gelatinised and lipids deteriorated in quality. The quality of resultant flour is not ideal for cosmetic use. Furthermore, current cosmetic flour ingredients seldom fulfil the natural cosmetic requirements such as those defined under the Ecocert Natural certification scheme or the European standard, COSMOS, where the use of irradiation, disinfection chemicals and the current hydrothermal treatments are not permitted treatments.

There is a need to obtain a high quality colloidal oatmeal, which does not compromise the microbial quality and does not utilise unapproved chemical treatments or irradiation to do so. Furthermore, there is a need for oat cereal products, which have improved oil retention and increased viscosity for use in cosmetic, food, and coating applications.

### Objects and Summary of the Invention

The object of the current method is to naturally modify the oat flour in order to reach controlled and minimal levels of gelatinisation resulting in improved characteristics in cosmetics and foods. This invention provides specific oat cereal products for use in food and cosmetic application and a method for their production. During processing raw materials, the microbial purity of the oat flour is naturally improved and the behaviour modified in a controlled manner resulting in flour which is adapted for cosmetic use. In addition the method according to the invention ensures that the endogenous enzymes within the oats, are effectively neutralised. This enhances the shelf life of the resultant ingredient and in the corresponding cosmetic product.

It is already known that an extruder improves the microbial purity of the flours (Cheftel 1998), and in addition to that, an extruder, expander or a similar heating screw is a usual means to control the pathogens e.g. salmonella, in animal feeds. However these methods ignore major changes to flour structure and starch gelatinisation since the nutritional content is the important factor in animal feed. This invention presents an extruded oat flour product, where the microbial purity has been significantly improved and the behaviour of the flour is controllably changed. Thus the flour is therefore adapted for cosmetic and food use.

This invention also describes the effect of extrusion on the enforced colloidal behaviour, improving flour solubility and phytochemical (betaglucan, avenanthramides, and protein) availability.

These and other objects are achieved by the present invention as hereinafter described and claimed.

The first aspect of the invention is a colloidal oat flour product. The colloidal oat flour product is mainly characterized by that stated in the descriptive part of claim 1.

The second aspect of this invention is a method for producing a colloidal oat flour product. According to this invention the oat flour is extruded and finely milled. More specifically the method according to the present invention is mainly characterized by the description outlined in claim 7.

The third and fourth aspects of this invention are the uses of the colloidal oat flour products described here in food or cosmetic products or as a coating barrier in protective products.

The fifth aspect of the invention is a cosmetic formulation. The characteristic to said formulation is that it contains colloidal oat flour product(s) described here.

The preferred embodiments of the invention are disclosed in the dependent claims.

### Brief Description of the Drawings

**Figure 1****.** The figure shows curves of viscosity versus shear rate of the extruded and non-extruded colloidal oatmeal (dry matter at 10%).
**Figure 2****.** The photograph demonstrates the difference between the centrifuged colloidal oatmeal (dry matter 5 w %). The left test-tube is the control containing oatmeal that is conventionally milled and the right test-tube contains oatmeal that has been extruded and milled.
**Figure 3****.** The photographs (magnification 400x) show the iodine colourised colloidal oatmeal particles. The extruded version is on the left-hand side and the conventional flour on the right-hand side.

### Description of Preferred Embodiments

Colloidal oatmeal is specified in the United States Pharmacopeial Convention USP Monograph USP32 -NF27 page 2024, which specifically includes flour particle size. Whole grain oat flour together with the use of the extrusion process meets the specified characteristics for colloidal oatmeal, with the exception of particle size. Therefore the extruded and dried whole grain oat flour must be further milled to meet this requirement. The colloidal behaviour of flours requires the use of very small particle sizes in order for any cereal flour to achieve a colloidal state.

The extruded oat flour product of this invention has the following characteristics:
- The flour characteristics have been changed during extrusion in a controlled manner and the changes are slight in order to give flours adapted for cosmetic and food use.
- The extruded oat flour can be regarded natural or certified under Ecocert or COSMOS as 'natural'.
- The extruded oat flour can replace flour products, where in order to attain microbial purity unapproved methods such as irradiation have been used.

In one embodiment a colloidal oat flour product which has of at least 0.4%, preferably at least 0.6%, more preferably 0.8%, even more preferably 1.0% and most preferably at least 1.2% dissolved material as measured using the Brix method from 10 w -% water suspension. In this connection the Brix value indicates the approximate value of dissolved solids. The dissolved components include starch as well as beta-glucan, avenanthramides and flavonoids in a bioavailable form and the claimed product is thus improved compared to conventional colloidal flours.

In one embodiment a colloidal oat flour product is natural or capable of being certified by the certifying bodies, Ecocert or COSMOS as natural. In this connection "natural" means compliant with the COSMOS cosmetic and natural standards (Currently Version 1.1 dated 31^{st} January 2011), excluding processes such as ionizing radiation (Appendix III), and only approved ingredients of mineral origin (Appendix IV), or other permitted ingredients (Appendix V). There is increasing demand for certified natural products within both cosmetic and food applications.

In one embodiment an oat flour product has total microbial count less than 5000 cfu/g (measured from the sample as-is), preferably less than 3000 cfu/g, more preferably less than 1000 cfu/g, even more preferably less than 500 cfu/g, even more preferably less than 100 cfu/g, and most preferably less than 10 cfu/g. Microbial purity/quality is an essential feature in cosmetic and food applications.

In one embodiment the endogenous enzymes, specifically lipase, of the oat cereal product are destroyed in the extrusion process. Active cereal enzymes have an adverse effect on to the quality of the product and shelf life.

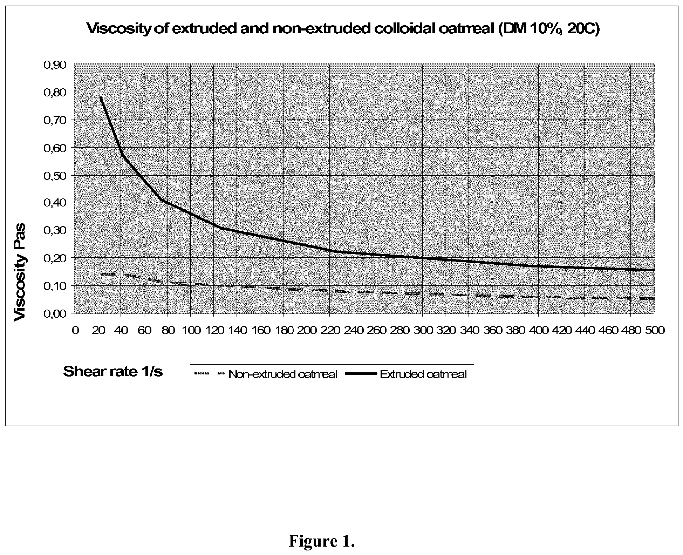

In one embodiment an oat flour product has viscosity of at least 0.150 Pas, preferably at least 0.170 Pas measured at 20 °C from 10% water suspension using a shear rate in the range of 20-40 l/s, preferably 22 to 25 l/s, which is the lowest shear rate in the Fig. 1. Enforced viscosity ensures that the product forms effective creams and lotions in cosmetic applications and is also suitable for food applications. Furthermore, such a product has an increased amount of free beta glucan, which is known to be an excellent film former as well as has the ability to penetrate the skin. (Pillai et al 2005).The oat flour product is of colloidal nature. The United States Pharmacopeial Convention for colloidal oatmeal also defines the maximum viscosity for colloidal oatmeal and the corresponding method of assay. The viscosity of 4.5% colloidal oatmeal must be less than 0.1 Pas. The method of measuring viscosity according to the monograph is described in the examples. In one embodiment of the invention the viscosity of 4.5% colloidal cereal product is be less than 0.1 Pas.

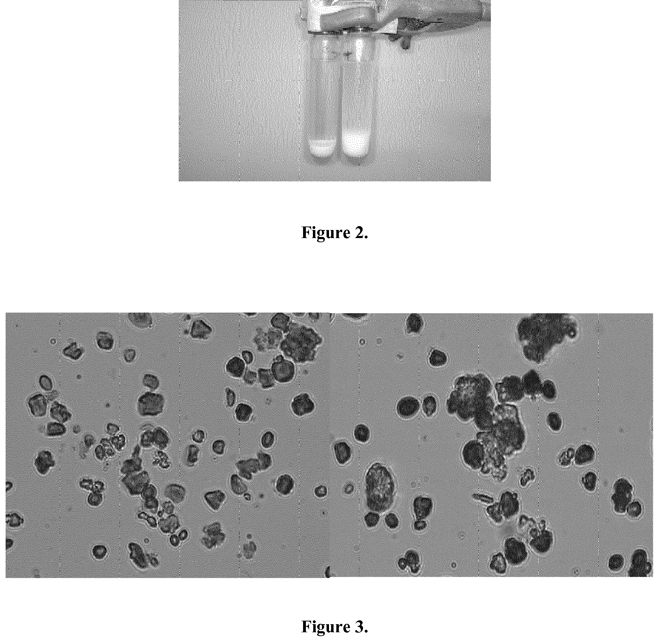

In one embodiment the oat cereal product has improved oil retention properties compared to non-extruded flour product. One reason for this is that extrusion releases amylose, which complexes and binds lipids (Fig. 3). This phenomenon is known to extend the shelf life of the products. The increased oil content gives improved skin feel in pressed powders e.g. eye shadow, as well as improving the moisturising properties of the oatmeal.The oat cereal product has reduced and modified particles, which improves the colloidal nature of the flour as well as improving skin feel. (Fig. 2)

In one embodiment the starch of the oat cereal product is slightly gelatinized in a controlled manner, whereas material made from non-extruded flour was not gelatinized. Partial gelatinization improves the solubility and maintains the colloidal nature of the product. Partial gelatinization also partially releases amylose.

Oats have increased levels of an endogenous polysaccharide, beta glucan. Beta glucan is known to have immunestimulent, moisturising and film forming properties. Small starch grains of oats, when compared to wheat, maize, barley and rye, are well adapted for cosmetic applications. The nutritional benefits of beta glucan have previously been discussed in detail e.g. in WO 2004/099257.

Oats also have a high oil content (compared to wheat, maize, barley and rye), which brings high levels of natural antioxidants and emulsifiers, thereby improving moisturisation and skin feel.

The extrusion method described here includes the extruder settings and the operational parameters, which together with the correct specification of raw material flour characteristics are suitable for producing colloidal oatmeal.

One embodiment of the invention is a method for producing a colloidal oat flour product that comprises the steps of extruding the oat flour and fine grinding of the extruded flour. According to the invention the conditions in extrusion chamber are controlled by
(a) maintaining the moisture below 17 w %, preferably below 15 w % and most preferably below 13 w %; and
(b) adding oil(s) into the feed at 1 to 6 w %, preferably 2 to 4 w % and most preferably about 3 w %; and/or
(c) lowering pressure of extrusion chamber by 20%, preferably 40% and even 60% compared to conventional pressure in chamber.

Usually the oil content in oats is suitable for the process, whereas other cereals have lower endogenous fat contents and thus require the addition of oil, typically vegetable oil. The oil component, which is added during extrusion, must be capable of sustaining temperatures of 120 °C without suffering any deterioration of quality during extrusion. If the oil concentration on entry to the extruder or expander exceeds 14 w % part of the oil may separate as a free phase.

One embodiment of the invention is a method that comprises the steps of extruding the oat cereal grain and fine grinding, wherein the pressure of the extrusion device is lowered under controlled conditions. The pressure of the extrusion chamber can be lowered by mechanical means e.g. widening the nozzles or removing the nozzle plate of the extruder. In a preferred embodiment the effective (back) pressure is created by the use of a reverse screw, with enlarged nozzles or without a nozzle plate.

The extrusion process efficiently kills the unwanted microbes in the feed without the need of additional treatments e.g. by irradiation or chemicals or the use of other hydrothermal methods such as pasteurization, UHT-treatment or autoclaving. Hydrothermal methods require large quantities of water resulting in uncontrolled gelatinization of starch. Irradiation or chemical treatments are not preferred treatment by some consumers. In this process the moisture during extrusion should be low in order to avoid over-gelatinization of the product.

Usually dehulled oats are stabilized by heat-treating whole dehulled kernels in order to destroy the endogenous enzyme, lipase. In the extrusion process described here wholemeal oat flour can be stabilized. This ensures that endogenous lipase and other enzymes located also in the core of the kernel are destroyed. A separate stabilization step is not therefore required which increases process economy.

Another benefit of the method of this invention is the partial release of amylose, which results in improved oil binding, enforced colloidal nature and more suitable viscosity.

These benefits have been already discussed above in connection with the colloidal oat flour product.

In one embodiment of this colloidal oat flour product invention is that it is used in preparation of a cosmetic product, which term is also intended to include toiletries compositions. The benefits of oats, and particular colloidal oatmeal has been known for some time (Kurtz E S et al 2007), and this invention enhances the properties of colloidal oatmeal by increasing oil binding and the colloidal properties giving an improved skin feel as well as enhanced soothing properties.

In another embodiment of this colloidal oat flour product invention it is used in preparation of a food product. The role of beta glucan in nutrition and health is discussed in other patents e.g. WO 2004/099257.

In still another embodiment the colloidal oat flour product of this invention is used as a coating barrier in protective products such as gloves, especially latex or nitrile gloves. The use of colloidal oatmeal as a coating on barrier protective gloves requires the oatmeal to have very low particulate concentration within the lining of the glove, and be capable of showing effective moisturisation, anti-irritancy or redness reduction properties at the defined oatmeal concentration of 0.007 w % [FDA Monograph Vol. 69, No. 160, 2004]. Neuser et al describe the benefits of colloidal oatmeal in patent US7691436B2. The colloidal oat flour product according to our invention enhances the solubility of the colloidal oatmeal thereby reducing particulate concentration, and as the solubility of the beta-glucan and other active molecules is improved through the process this enhances the moisturisation, anti-irritancy and redness reduction capabilities.

This invention also concerns cosmetic formulations containing the colloidal oat flour product described above. The cosmetic formulation has the following specific characteristics:
- The colloidal behaviour is enforced by extrusion
- The particle behaviour enforces the colloidal property
- The presense of beta glucan is maximised during extrusion
- The amylose fraction is partially freed during extrusion
- The cosmetic ingredient can be certified as natural

In an embodiment the cosmetic formulation contains a colloidal oat flour product described here, or a product obtained using a method as described here. The cosmetic formulation may be a balm, lotion, mask, shampoo, hair conditioner, soap, moisturizer, sunscreen, peeling cream, powder, without restricting to these.

The cosmetic and/or toiletries compositions (or formulations) of the present invention may contain 0.05 to 50% of the extruded colloidal oatmeal by weight of the total composition. In compositions intended to be applied topically to the skin to protect the skin the amount of extruded colloidal oatmeal that may be present is preferably in the range 0.5 to 10%, more preferably 1 to 6%.

The compositions of the present invention may contain a safe and effective amount of one or more inorganic and organic sunscreening agents

Suitable inorganic sunscreening agents include:
a) Microfine titanium dioxide;
b) Microfine zinc oxide; and
c) Boron nitride.

Examples of suitable additional organic sunscreening agents include:
a) para-aminobenzoic acids, esters and derivatives thereof, for example, 2-ethylhexyl para-dimethylaminobenzoate and the octyl ester of para-aminobenzoic acid;
b) methoxycinnamate esters such as 2-ethylhexyl para-methoxycinnamate, 2-ethoxyethyl para-methoxycinnamate or α,β-di-(para-methoxycinnamoyl)-α'-(2-ethylhexanoyl)-glycerin;
c) benzophenones such as oxybenzone;
d) dibenzoylmethanes such as 4-(tert-butyl-4'-methoxydibenzoylmethane;
e) 2-phenylbenzimidazole-5 sulfonic acid and its salts;
f) alkyl-β,β-diphenylacrylates for example alkyl α-cyano-β,β-diphenylacrylates such as octocrylene;
g) triazines such as 2,4,6-trianilino-(p-carbo-2-ethyl-hexyl-1-oxi)-1,3,5 triazine; and
h) camphor derivatives such as methylbenzylidene camphor.
i) organic pigments sunscreening agents such as methylene bis-benzotriazxole tetramethyl butylphenol
j) silicone based sunscreening agents such as dimethicodiethyl benzal malonate

The sunscreening agents of the present invention may be incorporated into sunscreen products such as oil phase dispersions or emulsions in the conventional way. The emulsion may be an oil-in-water emulsion:
The oil phase of the oil phase dispersions and the water-in-oil and oil-in-water emulsions of the present invention may comprise for example:
   a) hydrocarbon oils such as paraffin or mineral oils;
   b) waxes such as beeswax or paraffin wax;
   c) natural oils such as sunflower oil, apricot kernel oil, shea butter or jojoba oil;
   d) silicone oils such as dimethicone, cyclomethicone or cetyldimethicone;
   e) fatty acid esters such as isopropyl palmitate, isopropyl myristate or dioctylmaleate;
   k) fatty alcohols such as cetyl alcohol or stearyl alcohol; or
   g) mixtures thereof, for example, the blend of waxes available commercially under the trade name Cutina (Henkel).

In preferred water-in-oil compositions of the present invention the oil phase comprises 5 to 40%, more preferably 10 to 30% by weight of the composition. In preferred oil-in-water compositions of the present invention the oil phase comprises 5 to 30%, more preferably 10 to 20% by weight of the composition.

The emulsifiers used may be any emulsifiers known in the art for use in water-in-oil or oil-in-water emulsions. It has been found that particularly effective water-in-oil and oil-in-water sunscreen compositions can be prepared by using an emulsifier or mixture of emulsifiers selected from known cosmetically acceptable emulsifiers which include; sesquioleates such as sorbitan sesquioleate, or polyglyceryl-2-sesquioleate; ethoxylated esters of derivatives of natural oils such as the polyethoxylated ester of hydrogenated castor oil; silicone emulsifiers such as silicone polyols; anionic emulsifiers such as fatty acid soaps e.g. potassium stearate and fatty acid sulphates e.g. sodium cetostearyl sulphate; ethoxylated fatty alcohols; sorbitan esters; ethoxylated sorbitan esters; ethoxylated fatty acid esters such as ethoxylated stearates,; ethoxylated mono-, di-, and tri-glycerides; non-ionic self-emulsifying waxes; ethoxylated fatty acids; mixtures thereof

The compositions of the present invention may additionally comprise other components which will be well known to those skilled in the art. These include, for example, emolients such as isopropyl myristate or triglycerides of fatty acids e.g. lauric triglyceride or capric/caprylic triglyceride, such as the triglyceride available commercially under the trade name Migliol 810 (Huls UK); moisturisers such as D-panthenol; humectants such as glycerin or 1,3-butylene glycol; antioxidants such as DL-α-tocopherylacetate or butylated hydroxytoluene; emulsion stabilising salts such as sodium chloride, sodium citrate or magnesium sulphate; film formers to assist spreading on the surface of the skin such as alkylated polyvinylpyrrolidone e.g. available commercially under the trade name Antaron (GAF); thickeners such as acrylic acid polymers e.g. available commercially under the trade name Carbopol (B.F. Goodrich) or modified celluloses e.g. hydroxyethylcellulose available commercially under the trade name Natrosol (Hercules) or alkylgalactomanans available under the trade name N-Hance; preservatives such as bronopol, sodium dehydroacetate, polyhexamethylenebiguanide hydrochloride, isothiazolone or diazolidinylurea; sequestering agents such as EDTA salts; perfumes and colourings.

The compositions of the present invention may also contain a safe and effective amount of one or more anti-acne actives. Examples of useful anti-acne actives include resorcinol, sulfur, salicylic acid, benzoyl peroxide, erythromycin, zinc, etc.

The compositions of the present invention may further contain a safe and effective amount of one or more anti-wrinkle actives or anti-atrophy actives. Exemplary antiwrinkle/anti-atrophy actives suitable for use in the compositions of the present invention include sulfur-containing D and L amino acids and their derivatives and salts, particularly the N-acetyl derivatives, a preferred example of which is N-acetyl-L-cysteine; thiols, e. g. ethane thiol; hydroxy acids (e. g., alpha-hydroxy acids such as lactic acid and glycolic acid or beta-hydroxy acids such as salicylic acid and salicylic acid derivatives such as the octanoyl derivative), phytic acid, lipoic acid; lysophosphatidic acid, skin peel agents (e. g., phenol and the like), vitamin B compounds and retinoids which enhance the keratinous tissue appearance benefits of the present invention, especially in regulating keratinous tissue condition, e. g., skin condition.

The compositions of the present invention may also contain a retinoid. As used herein, "retinoid"includes all natural and/or synthetic analogs of Vitamin A or retinol-like compounds which possess the biological activity of Vitamin A in the skin as well as the geometric isomers and stereoisomers of these compounds. The retinoid is preferably retinol, retinol esters (e. g., C2 -C22 alkyl esters of retinol, including retinyl palmitate, retinyl acetate, retinyl propionate), retinal, and/or retinoic acid (including all-trans retinoic acid and/or 13-cis-retinoic acid), more preferably retinoids other than retinoic acid.

Additional peptides, including but not limited to, di-, tri-, tetra, penta and hexapeptides and derivatives thereof, may be included in the compositions of the present invention in amounts that are safe and effective. As used herein,"peptides" refer to both the naturally occurring peptides and synthesized peptides. Also useful herein are naturally occurring and commercially available compositions that contain peptides.

The compositions of the present invention may include a safe and effective amount of an anti-oxidant/radical scavenger. The anti-oxidant/radical scavenger is especially useful for providing protection against UV radiation which can cause increased scaling or texture changes in the stratum corneum and against other environmental agents which can cause skin damage.

A safe and effective amount of an anti-oxidant/radical scavenger may be added to the compositions of the subject invention, preferably from about 0.1% to about 10%, more preferably from about I % to about 5%, of the composition.

Anti-oxidants/radical scavengers such as ascorbic acid (vitamin C) and its salts, ascorbyl esters of fatty acids, ascorbic acid derivatives (e. g., magnesium ascorbyl phosphate, sodium ascorbyl phosphate, ascorbyl sorbate), tocopherol (vitamin E), tocopherol sorbate. tocopherol acetate, other esters of tocopherol. butylated hydroxy benzoic acids and their salts, 6-hydroxy acid (commercially available under the tradename TroloxR), gallic acid and its alkyl esters, especially propyl galate, uric acid and its salts and alkyl esters, sorbic acid and its salts, esters of tocopherol, lipoic acid, amines (e. g., N, N-diethylhydroxylamine, amino-guanidine), sulfhydryl compounds (e. g., glutathione), dihydroxy fumaric acid and its salts. lycine pidolate, arginine pilolate, nordihydroguaiaretic acid, bioflavonoids, curcumin,lysine, methionine, proline, superoxide dismutase, silymarin, tea extracts, grape skin/seed extracts, melanin, and rosemary extracts may be used.

The compositions of the present invention may also contain a safe and effective amount of a chelator or chelating agent. As used herein, "chelator" or "chelating agent" means an active agent capable of removing a metal ion from a system by forming a complex so that the metal ion cannot readily participate in or catalyze chemical reactions. The inclusion of a chelating agent is especially useful for providing protection against UV radiation which can contribute to excessive scaling or skin.

Flavonoids suitable for use in the present invention are flavanones; chacones; isoflavones; coumarins; chromones ; one or more dicoumarols; one or more chromanones; one or more chromanols;

A safe and effective amount of an anti-inflammatory/soothing agent may be added to the compositions of the present invention, These include Steroidal anti-inflammatory agents, including but not limited to, corticosteroids and nonsteroidal anti-inflammatory agents, and also so-called"natural"anti-inflammatory agents for example bisabolol, aloe vera, plant sterols , kola extract, chamomile, red clover extract, and compounds of the Licorice (the plant genus/species Glycvrrhiza glabra) family, including glycyrrhetic acid, glycyrrhizic acid, and derivatives thereof (e. g., salts and esters).

The compositions of the present invention may also contain a safe and effective amount of an anti-cellulite agent such as xanthine compounds (e. g., caffeine, theophylline, theobromine, and aminophylline).

The compositions of the present invention may also contain a safe and effective amount of a topical anesthetic. Examples of topical anesthetic drugs include benzocaine, lidocaine, bupivacaine, chlorprocaine, dibucaine, etidocaine, mepivacaine, tetracaine, dyclonine, hexyl- caine, procaine, cocaine, ketamine, pramoxine, phenol, and pharmaceutically acceptable salts thereof.

The compositions of the present invention may contain a tanning active such as dihydroxyacetone or erythrulose as an artificial tanning active.

The compositions of the present invention may contain a skin lightening agent including kojic acid, arbutin, ascorbic acid and derivatives thereof (e. g., magnesium ascorbyl phosphate or sodium ascorbyl phosphate), and extracts (e. g., mulberry extract, placenta extract).

The compositions of the present invention may contain an antimicrobial or antifungal active. Examples of antimicrobial and antifungal actives include B-lactam drugs, quinolone drugs, ciprofloxacin, norfloxacin, tetracycline, erythromycin. amikacin, 2,4,4'-trichloro-2'hydroxy diphenyl ether, 3,4,4'-trichlorobanilide, phenoxyethanol, phenoxy propanol, phenoxyisopropanol, doxycycline, capreomycin, chlorhexidine. chlortetracycline, oxytetracycline, clindamycin, ethambutol. hexamidine isethionate, metronidazole, pentamidine, gentamicin, kanamycin, lineomycin, methacycline, methenamine, minocycline, neomycin, netilmicin, paromomycin, streptomycin, tobramycin, miconazole, tetracycline hydrochloride, erythromycin, zinc erythromycin, erythromycin estolate, erythromycin stearate, amikacin sulfate, doxycycline hydrochloride, capreomycin sulfate, chlorhexidine gluconate, chlorhexidine hydrochloride, chlortetracycline hydrochloride, oxytetracycline hydrochloride, clindamycin hydrochloride, ethambutol hydrochloride, metronidazole hydrochloride, pentamidine hydrochloride, gentamicin sulfate, kanamycin sulfate, lineomycin hydrochloride, methacycline hydrochloride, methenamine hippurate, methenamine mandelate, minocvcline hydrochloride, neomycin sulfate, netilmicin sulfate, paromomycin sulfate, streptomycin sulfate, tobramycin sulfate, miconazole hydrochloride, ketaconazole, amanfadine hydrochloride. amanfadine sulfate, octopirox, parachlorometa xylenol, nystatin, tolnaftate, zinc pyrithione and clotrimazole.

Preferred examples of actives useful herein include those selected from salicylic acid, benzoyl peroxide, 3-hydroxy benzoic acid, glycolic acid, lactic acid, 4-hydroxy benzoic acid, acetyl salicylic acid, 2-hydroxybutanoic acid, 2-hydroxypentanoic acid. 2-hydroxyhexanoic acid, cis-retinoic acid, trans-retinoic acid, retinol, phytic acid, N-acetyl-L-cysteine, lipoic acid, azelaic acid, arachidonic acid, benzoylperoxide, tetracycline, ibuprofen, naproxen, hydrocortisone, acetominophen, resorcinol, phenoxyethanol, phenoxypropanol, phenoxyisopropanol, 2,4,4'-trichloro-2'-hydroxy diphenyl ether, 3,4,4'-trichlorocarbanilide, octopirox, lidocaine hydrochloride, clotrimazole, miconazole, ketoconazole, neocycin sulfate, and mixtures thereof.

The compositions of the present invention may contain a particulate materials such as; bismuth oxychloride, iron oxide, mica, mica treated with barium sulfate and Ti02, silica, nylon, polyethylene, talc, styrene, polyproylene. ethylene/acrylic acid copolymer, sericite. aluminum oxide, silicone resin, barium sulfate, calcium carbonate, cellulose acetate, titanium dioxide, polymethyl methacrylate, and mixtures thereof.

The compositions of the present invention may contain a conditioning agent selected from humectants, moisturizers, or skin conditioners. These materials include, but are not limited to, guanidine; urea; glycolic acid and glycolate salts (e. g. ammonium and quaternary alkyl ammonium); salicylic acid; lactic acid and lactate salts (e. g., ammonium and quaternary alkyl ammonium); aloe vera in any of its variety of forms (e. g., aloe vera gel); polyhydroxy alcools such as sorbitol, mannitol, xylitol, erythritol, glycerol, hexanetriol, butanetriol, propylene glycol, butylene glycol, hexylene glycol and the like; polyethylene glycols; sugars (e. g., melibiose) and starches; sugar and starch derivatives (e. g., alkoxylated glucose, fructose, glucosamine); hyaluronic acid; lactamide monoethanolamine; acetamide monoethanolamine; panthenol; allantoin; and mixtures thereof.

The compositions of the present invention can contain one or more thickening and structuring agents such as, Carboxylic Acid Polymers (Carbomers), Crosslinked Polyacrylate Polymers , Polyacrylamide Polymers, Polysaccharides such as carboxymethyl hydroxyethylcellulose, cellulose acetate propionate carboxylate, hydroxyethylcellulose, hydroxyethyl ethylcellulose, hydroxypropylcellulose, hydroxypropyl methylcellulose, methyl hydroxyethylcellulose, microcrystalline cellulose, sodium cellulose sulfate, and mixtures thereof, and Gums such as acacia, agar, algin, alginic acid, ammonium alginate, amylopectin, calcium alginate, calcium carrageenan, carnitine, carrageenan, dextrin, gelatin, gellan gum, guar gum, guar hydroxypropyltrimonium chloride, hectorite, hyaluroinic acid, hydrated silica, hydroxypropyl chitosan, hydroxypropyl guar, karaya gum, kelp, locust bean gum, natto gum, potassium alginate, potassium carrageenan, propylene glycol alginate, sclerotium gum, sodium carboyxmethyl dextran, sodium carrageenan, tragacanth gum, xanthan gum, and mixtures thereof.

The cosmetic formulation containing the colloidal oat flour product described herein has improved water retention, oil binding, pH buffering and moisturizing properties. The process allows the product to be certified as Natural (Ecocert), whereas traditional processes cannot make this claim.

In one embodiment the cosmetic formulation is a bath powder containing 10 to 60 %, preferably about 45w % of a colloidal oat flour product described here or prepared as described here. Preferably the colloidal oat flour product is colloidal oatmeal (INCI: Avena sativa Kernel Meal). In one embodiment the formulation also contains oils, starch, silica and laureth-4.

In one embodiment the cosmetic formulation is a baby balm, face mask or body lotion containing 1 to 10 w -% of a colloidal oat flour product described here or prepared as described here.

Also combinations of the above described colloidal oat flour product and other active ingredients are included in the scope of the invention. These can be used, for example, in products for sun care, rosacea, specific anti-ageing etc.

Such combination products can comprise:
a) a safe and effective amount, preferably from 50 to 0.1%, of a colloidal oat flour based active selected from the group consisting of extruded colloidal oatmeals and derivatives thereof,
   and mixtures thereof;
b) a safe and effective amount of at least one additional active; and
c) a dermatologically acceptable carrier.

The additional active is preferably selected from the group consisting of desquamatory actives, anti-acne actives, vitamin B compounds, retinoids, peptides, hydroxy acids, antioxidants, radical scavengers, chelators, antiinflammatory agents, topical anesthetics, tanning actives, skin lightening agents, anti-cellulite agents, anti-wrinkle actives, flavonoids, antimicrobial actives, skin soothing agents, skin healing agents, antifungal actives, sunscreen actives, conditioning agents, structuring agents, thickening agents, herbal extracts, and mixtures thereof.

### Examples of colloidal oatmeal

### Example 1. Whole grain oat flour extrusion in a pilot extruder

Whole grain oat flour (dry matter 87%) was extruded using a Clextral BC21. The retention time in the extruder was 31 seconds. The feed was at 4.6 kg/h and the extruder screw speed at 80 rpm. Extruder temperature was maintained along the extruder barrel at 110°C to 120°C. The extruder did not have a nozzle plate or nozzles in order to decrease the energy consumption and torque during extrusion. The pressure in the extruder was maintained by the use of a reverse screw at the end of the barrel. The pressure generated by the reverse screw was high enough to keep the water in the liquid state without steam formation, a phenomenon which enhances the destruction of microbes.

The extrusion of whole grain oat flour without a nozzle plate and nozzles gave surprising results as shown in **Table 1**. The conditions in the extrusion also without added water were effective enough to kill the microbes existing in the whole grain oat flour.

**Table 1.**

| | **Temp. [°C]** | **Inlet moisture w %** | **Outlet moisture w %** | **Water activity Aw** | **Microbes cfu/g** | **Yeast cfu/g** | **Moulds cfu/g** |
|---|---|---|---|---|---|---|---|
| Flour | | 13% | - | 0.6 | 7x10⁵ | 5400 | 730 |
| Test 1 | 120 | 13% | 7.5% | 0.4 | <10 | <10 | <10 |
| Test 2 | 120 | 19% | 12.2% | 0.6 | <10 | <10 | <10 |
| Test 3 | 110 | 13% | 7.2% | 0.3 | <10 | <10 | <10 |
| Test 4 | 110 | 19% | 11.9% | 0.6 | <10 | <10 | <10 |

### Comparative Example 2. Whole grain wheat flour extrusion in a pilot extruder

Whole grain wheat flour (dry matter 87.7 w %) with 5 w % added sunflower oil was extruded using a Clextral BC21 extruder. The retention time was 31 seconds. The feed rate was 4.6 kg/hour and the extruder screw speed at 80 rpm. Extruder temperature was maintained at 120°C throughout the barrel. The nozzle plate and nozzles were not fitted to the extruder in order to decrease the energy consumption and torque. The pressure in the extruder was maintained by the reverse screw and was of a smaller pitch that that used in oat flour test procedure. The pressure generated by the reverse screw was high enough to keep the water in the liquid state without steam formation, a phenomenon which enhances the destruction of microbes.

The extrusion of whole grain wheat flour without a nozzle plate and nozzles gave surprising results as shown in **Table 2**. The conditions in the extrusion without added water were effective enough to kill the microbes existing in the whole grain wheat flour.

**Table 2.**

| | **Temp. [°C]** | **Inlet moisture w%** | **Outlet moisture w%** | **Microbes cfu/g** | **Yeast cfu/g** | **Moulds cfu/g** |
|---|---|---|---|---|---|---|
| Flour | | | 12.3% | 8x10³ | <100 | <100 |
| Test 1 | 120 | 12.3% | 8.2% | <10 | <10 | <10 |

### Example 3. Whole grain oat flour extrusion in an industrial scale extruder

Whole grain oat flour (dry matter 86.8%) was extruded using a Clextral BC72 extruder. The retention time was 25 seconds and the feed was 304 kg/hour with an extruder screw speed of 130 rpm. The extruder temperature was equalised along the barrel at 120 °C excluding the first section. The extruder did not have a nozzle plate or nozzles fitted in order to decrease the energy consumption and enhance passage through the barrel during extrusion. Pressure in the extruder was maintained by the reverse screw at the barrel exit. The pressure generated by the reverse screw was high enough to keep the water in the liquid state without steam formation, a phenomenon which enhances the destruction of microbes. The extruded products were conditioned (heater-cooler) in a belt drier and fine milled to a finished product. The microbial samples were taken after the conditioner. Analysis results are shown in **Table 3**.

**Table 3.**

| | **Temp. [°C]** | **Inlet moisture w %** | **Outlet moisture w %** | **Microbes cfu/g** | **Yeast cfu/g** | **Moulds cfu/g** |
|---|---|---|---|---|---|---|
| Flour | | 13.0% | - | 7x10⁵ | 5400 | 730 |
| Test 1 | 120 | 13.0% | 9.4% | <10 | <10 | <10 |

The conditions in the industrial scale extrusion without added water were effective enough to kill the microbes existing in the whole grain oat flour.

### Example 4. Comparison of viscosities of extruded and non-extruded colloidal oatmeal measured against shear rate.

Extruded whole grain oat flour (Example 3.) was fine milled (Görgens, Turborotor G-55) on an industrial scale in order to reach the colloidal oatmeal specification (The United States Pharmacopeial Convention USP 32 -NF27). For comparison standard oat flakes were fine milled with the same mill in order to reach the same colloidal oatmeal specification. The relative viscostatic behaviours were compared to each other. The viscosity/shear rate curves of extruded and non-extruded colloidal oatmeal were measured with a Bohlin 88 viscometer with the measuring head C 25 (spindle cylinder 25 mm, outer cylinder 27.5 mm) enl. DIN 53019. Dry matter was 10 w % and temperature 20°C. The results of viscosities versus shear rate are shown in Figure 1.

Both oatmeals had non-Newtonian viscosity behaviour, which demonstrates that they had the highest viscosities at the low shear rate and lowest viscosities at the highest shear rate. This viscosity behaviour is associated with soluble betaglucan. We confirmed that the viscosity was largely caused by betaglucan. A betaglucanase enzyme (Econase CE, AB Enzymes) was added to both oatmeal suspensions at 10 w % dry matter, which decreased the viscosity immediately to a level that corresponds to water and was not measurable with the Bohlin 88 instrument. Alpha-amylase was also added, which had no effect on the viscosities.

The viscosity of extruded colloidal oatmeal was significantly higher than the viscosity of non-extruded oatmeal. The higher viscosity of extruded version showed that it had increased dissolved beta glucan than non-extruded version. We evaluated the dissolved beta glucan concentrations of extruded and non-extruded oatmeal suspensions at 10 w % dry matter. Brix-values of centrifuged clear liquids of both samples were measured, as in this connection the Brix assay indicates an approximate concentration of dissolved solids. The Brix value of extruded version was 1.2 (1.2 w %) and non-extruded version 0.4 (0.4 w %). We know that dehulled Finnish oat varieties contain approximately 5 w % betaglucan (dry matter basis), which indicates that the highest dissolved concentration of betaglucan in oatmeal suspension at 10 w % dry matter is approximately 0.5 w %. A significant part of the difference of the dissolved solid concentrations (0.8 w %) can be attributed to the beta glucan concentration. Maunsell et al (2011) have observed the beneficial effects of betaglucan in cosmetic applications.

The viscosity difference between the extruded and non-extruded versions was highest, at the point of lowest shear rate. As result of this the viscosity measured at the lowest shear rate demonstrates the best effect of the extrusion process on maximising the availability of the beta glucan in the colloidal oatmeal. In this example the lowest shear rate was 23.3 l/s, which was achieved, when stirring rate of the spindle was 20 rpm.

### Example 5. The viscosity upper limit of 0.1 Pas defined by USPC Monograph USP 32 -NF27: measured from extruded and non-extruded colloidal oatmeal

The viscosities were measured according to the method defined in the United States Pharmacopeial Convention. A 25 g sample was added to 500 ml of water in small portions whilst being stirred at 1000 rpm over 1 minute. This procedure results in a flour suspension where the dry matter concentration was 4.5 w %. The starting temperature of the water was 45°C and it was maintained at this temperature throughout the mixing stage. Stirring was continued for 5 minutes after the addition of the last portion of oatmeal. The suspension was left to stand for 90 minutes and equalise to ambient temperature. The suspension was stirred at 800 rpm for 1 minute. The viscosity was then measured using a Brookfield viscometer (DV-II+) fitted with spindle no 1 and set to 60 rpm. The spindle 1 has a cylinder 1.88cm in diameter and 6.25cm high attached to shaft of 0.32cm in diameter, the distance from the top of the cylinder to the lower tip of the shaft being 0.75cm and the immersion depth being 8.15cm. This arrangement outlines the geometry when the viscosity is measured, and creates repeatable conditions without defining the exact shear rate. Both the extruded version and non-extruded version had lower viscosities than 0.1 Pas.

### Example 6. Colloidal behaviour of the extruded and non-extruded colloidal oatmeal

The colloidal oatmeal samples were mixed to 5 w % suspension and centrifuged in a laboratory centrifuge (3000 rpm, 10 min). The tubes were then photographed. The non-extruded version is on the left and extruded version on the right. The photo is presented in Figure 2.

The non-extruded version has clear layers and smaller layer of solids on the bottom. The extruded version has a hazy top layer and more solids on the bottom. This behaviour indicates that the extruded version has enforced colloidal behaviour.

### Example 7. Comparison of the particles of extruded and non-extruded colloidal oatmeal

Extruded whole grain oat flour (Example 3) was fine milled in an industrial mill to the colloidal oatmeal specification (United States Pharmacopeial Convention USP 32 -NF27). Standard oat flakes were fine milled in the same mill in order to reach the same colloidal oatmeal specification. Particles of the both colloidal oatmeals were stained with Lugol-iodine solution and the particles photographed under a microscope (Figure 3). The extruded version is on the left and non-extruded version on the right.

The pictures show the extruded version may have more damaged particles. The most significant indication is however that the extruded material has increased blue colour and the non-extruded, a darker violet colour. The blue colour indicates that the amylose fraction is more freely available in the extruded version. Extrusion is known to increase the amylose-lipid complexes, which will extend the shelf life of the lipids and therefore would show the same effect in finished products (Asp and Björck 1998)

### Example 8. Comparison of oil binding capacities of extruded and non-extruded colloidal oatmeal

30 grams of rapeseed oil (30g) was added to 5 grams of the two variants of oatmeals and placed in ultracentrifuge tubes. All samples were thoroughly mixed for 45 seconds to achieve a fully homogeneous state. After allowing the samples to rest for 30 min the samples were centrifuged at 5000 rpm for 30 min. The separated oil was decanted and the precipitate weighed. The increase of the weight of the precipitate (oatmeal) was calculated. The oil binding capacity was expressed as grams of rapeseed oil bound by 100g of oatmeal. The experiment was performed three times. Oatmeals were used in an as-is state with moisture contents of 5.4 and 7.9 w % respectively for native and extruded oatmeals. The results are shown in the table below.

**Table 4.**

| **Sample** | **Weight increase (g)** | **Average (g)** | **Oil binding (g/100g as is)** | **Oil binding (g/100g DM)** |
|---|---|---|---|---|
| Extruded oatmeal sample 1. | 3.89 | 3.9 | 78 | 85 |
| Extruded oatmeal sample 2. | 3.91 | | | |
| Extruded oatmeal sample 3. | 3.92 | | | |
| Native oatmeal sample 1. | 3.02 | 3.0 | 60 | 63 |
| Native oatmeal sample 2. | 2.98 | | | |
| Native oatmeal sample 3. | 3.02 | | | |

Extrusion increased the oil binding of the dry matter of oatmeal by 35% i.e. the oil binding capacity of the extruded oatmeal was significantly higher than that of the standard oatmeal.

### Examples of cosmetic formulations containing colloidal oatmeal

### Example 9. Soothing Natural Baby Balm (Formulation 1)

Whole grain oat flour was extruded (example 3) and fine milled (example 4) in order to reach the colloidal oatmeal specification (United States Pharmacopeial Convention USP 32 -NF27). The product was tested in Soothing Natural Baby Balm (Oat Services UK, JB Code OS BB1).

| **Phase** | **Common / Trade Name** | **Supplier** | **INCI Name** | **% w/w** |
|---|---|---|---|---|
| A | Water | | Aqua / Water | 30.1 |
| | Rheocare XG | BASF | Xanthan Gum | 0.40 |
| | Glycerin (vegetable) | | Glycerin | 5.00 |
| | Geogard Ultra | Lonza | Gluconolactone (and) Sodium Benzoate | 1.00 |
| | | | | |
| B | Sunflower Oil | A&E Connock | Helianthus Annuus Seed Oil | 30.00 |
| | Castor Oil | A&E Connock | Ricinus communis (Castor) Seed Oil | 10.00 |
| | Sisterna A10E-C | Sisterna | Sucrose Tetrastearate Triacetate | 3.50 |
| | Illipe Butter, Natural | Beraca | Shorea Stenoptera Seed Butter | 10.00 |
| | Lexgard GMCY | Inolex | Glyceryl Caprylate | 1.00 |
| | | | | |
| C | Sisterna SP70 C | Sisterna | Sucrose Stearate | 3.50 |
| | Dermofeel Toco 70 Non GMO | Dr Straetmans | Tocopherol, Helianthus Annuus (Sunflower) Seed Oil | 0.50 |
| | | | | |
| D | Colloidal Oatmeal | Oat Cosmetics | Avena Sativa Kernel Meal | 5.00 |
| | | | | |

Method of Manufacture: Phase A was heated to 50 °C and blended by stirring. Phase B was heated to 50 - 60 °C until it melted. Phase C was added to the heated phase B and mixed until homogeneous. Phase A was added to phase B/C with a high shear mixer. Once fully mixed, the colloidal oatmeal was added under homogenisation. Once the colloidal oatmeal was fully dispersed, and the resultant balm was poured into containers and allowed to cool.
The thick, soft balm leaves a silky but protective film on the baby's skin. The natural emulsion utilises the increased soothing benefits of colloidal oatmeal to help protect against skin issues such as diaper rash and help reduce any redness or irritation.

The product has an excellent cosmetic appearance, being a light ivory stable balm with a powdery and non-greasy after-feel on the skin.

### Example 10. Soothing Oatmeal and Lavender Bath Powder (Formulation 2)

Whole grain oat flour was extruded (example 3) and fine milled (example 4) in order to reach colloidal oatmeal specification (United States Pharmacopeial Convention USP 32 - NF27). The product was tested in a soothing oatmeal and lavender bath powder (Oat Services UK, JB Code OS BP1).

**Table 5.**

| **Phase** | **Common / Trade Name** | **Supplier** | **INCI Name** | **% w**/**w** |
|---|---|---|---|---|
| A | Sunflower Oil | A&E Connock | Helianthus Annuus Seed Oil | 20.00 |
| | Natrasorb Bath | Akzo Nobel | Tapioca Starch | 28.00 |
| | Procol LA-4 | Protameen Chemicals | Laureth-4 | 5.00 |
| | Colloidal Oatmeal | Oat Cosmetics | Avena Sativa Kernel Meal | 45.00 |
| B | Lavender Essential Oil | S&D Aroma | Lavandula Angustifolia (*Lavender*) *Oil* | 0.60 |
| | Aerosil 200 | Evonik Industries | Silica | 1.40 |

Method of Manufacture: The sunflower oil and Procol LA-4 of phase A were blended together. Natrasorb Bath was added and mixed thoroughly. The colloidal oatmeal was then added and mixed thoroughly. The lavender essential oil was blended with the Aerosil 200 of phase B and once homogeneous mixed into phase A. The complete powder was mixed until entirely homogenous and smooth.

This powder product is suitable for packaging in small single use sachets or "tea bags". The colloidal powder can easily be added directly to the bath water, or the tea bag is steeped in the warm water, to provide a soothing and moisturising effect. The colloidal oatmeal gives improved release of the beneficial active components, whilst the sunflower oil is effectively emulsified into the water to provide the additional moisturising properties of bath oil without any associated greasiness. Finally the lavender oil provides a pleasant odour with an aromatherapy calming benefit.

The colloidal oatmeal helps to absorb the oils and surfactant and provide a suitably dry powder. This could also be used in the shower as a gentle body exfoliator that is rinsed off, leaving soft, soothed skin.

### Example 11. Purifying and Moisturising Face Mask (Formulation 3)

Whole grain oat flour was extruded (example 3) and fine milled (example 4) in order to reach colloidal oatmeal specification (United States Pharmacopeial Convention USP 32 - NF27). The product was tested in a purifying and moisturising face mask (Oat Services UK, JB Code OS CM2).

**Table 6.**

| **Phase** | **Common / Trade Name** | **Supplier** | **INCI Name** | **% w/w** |
|---|---|---|---|---|
| A | Keltrol CG-SFT | CP Kelco | Xanthan Gum | 0.50 |
| | Water | | Aqua | 46.00 |
| | Glycerine (veg) | | Glycerin | 5.00 |
| | Dermosoft PEA eco | Dr Straetmans | Phenethyl Alcohol | 1.00 |
| | | | | |
| B | Pretiox AV-01-FG | Precolor | Titanium Dioxide | 1.00 |
| | | | | |
| C | Dermorganics GMS-SE | Dr Straetmans | Glyceryl Stearate SE | 6.00 |
| | Sunflower Oil | A&E Connock | Helianthus Annuus Seed Oil | 5.00 |
| | Lexgard GMCY | Inolex | Glyceryl Caprylate | 0.50 |
| | | | | |
| D | Amazonian White Clay RFAWC 3900 | Beraca | Kaolin | 10.00 |
| | Colloidal Oatmeal | Oat Cosmetics | Avena Sativa Kernel Meal | 5.00 |
| | Rose Water | Zahra Rosewater Co. | Rosa Damascena (Rose) Flower Distillate | 10.00 |
| | | | | |
| E | | | | |
| | Grain Alcohol | | Ethanol Denat. | 10.00 |

Method of Manufacture: Xanthan gum was dispensed in the water of phase A by stirring. Phase A was heated to 75-80 °C. Phase B was added and mixed until homogeneous. Phase C was heated to 75-80 °C and Phase A/B added using a propeller stirrer. The resultant mixture was homogenised whilst phase D was added to the batch. The batch was then cooled to room temperature whilst under sweep agitation. Phase E was added when the temperature was below 40 °C.

The creamy face mask is applied to the skin and allowed to dry before rinsing off with tepid water. The emulsion base contains both colloidal oatmeal to soothe and calm, along with clay to draw impurities out of the skin. The mask is suitable for those people who find simple clay masks too astringent and require a more hydrating and soothing option.

The colloidal oatmeal provides skin care benefits whilst not interfering with the appearance or drying of the mask and providing a soft skin feel.

### Example 12. All Natural After-sun Body Lotion (Formulation 4)

Whole grain oat flour was extruded (example 3) and fine milled (example 4) in order to reach colloidal oatmeal specification (United States Pharmacopeial Convention USP 32 - NF27). The product was tested in all natural after-sun body lotion (Oat Services UK, JB Code OC OM 3.1).

**Table 7.**

| **Phas e** | **Common / Trade Name** | **Supplier** | **INCI Name** | **% w/w** |
|---|---|---|---|---|
| A | Water | | Aqua / Water | 74.10 |
| | Glycerin | | Glycerin | 5.00 |
| | Dermosoft 1388 Eco (preservative) | Dr Straetmans | Glycerin, Aqua, Sodium Levulinate, Sodium Anisate | 3.00 |
| | Dermofeel PA-3 | Dr Straetmans | Sodium Phytate, Aqua | 0.10 |
| | Keltrol CG-RD | CP Kelco | Xanthan Gum | 0.20 |
| | | | | |
| B | Symbiomuls GC | Dr Straetmans | Glyceryl Stearate Citrate, Cetearyl Alcohol, Glyceryl Caprylate | 5.00 |
| | Apricot Kernel Oil | Azelis | Prunus Armeniaca (*Apricot*) *Kernel Oil* | 2.00 |
| | Jojoba Oil, Colourless | Desert Whale | Simmondsia Chinenesis (Jojoba) Seed Oil | 1.00 |
| | Dermofeel Sensolv | Dr Straetmans | Isoamyl Laurate | 3.00 |
| | Plantec Olive Squalane | CRM International | Squalane | 1.00 |
| | Copherol F1300 G | BASF | Tocopherol | 0.10 |
| | DL-Tocopheryl Acetate | DSM | Tocopheryl Acetate | 0.50 |
| | | | | |
| C | Trealix | Sinerga | Trehalose, Hydrolyzed Vegetable Protein | 2.00 |
| | Colloidal Oatmeal | Oat Cosmetics | Avena Sativa Kernel Meal | 3.00 |

Method of Manufacture: The xanthan gum was dispersed in the water, and when homogeneous the other ingredients of phase A were added. Phases A and B were heated separately to 78 °C. Phase B was added to A using a paddle stirrer and then homogenised for 1-2 minutes. Stirring and cooling was continued and phase C was then added when the mixture was below 45 °C. Stirring was continued until the mixture was cool.

This natural soft cream leaves a moisturising film on the skin. Colloidal oat flour helps to soothe the skin and provides a slightly powdery skin feel. (Kurtz and Wallo, 2007)

### Example 13. Aftersun Lotion (Formulation 5)

| | %w/w |
|---|---|
| Aqua | to 100 |
| Hydrated silica | 5 |
| Isopropyl palmitate | 4 |
| Arachidyl propionate | 2 |
| Dimethicone | 2 |
| Glycerin | 2 |
| Steareth-21 | 1.96 |
| Steareth-2 | 1.683 |
| Cetyl alcohol | 1 |
| Tribehenin | 1 |
| Glyceryl stearate | 1 |
| Paraffinum liquidum | 0.994 |
| Panthenol | 0.75 |
| Parfum | 0.3 |
| Xanthan gum | 0.3 |
| Sodium citrate | 0.25 |
| Tocopheryl acetate | 0.2 |
| Hydroxyethylcellulose | 0.1 |
| Bisabolol | 0.095 |
| Citric acid | 0.05 |
| Preservative | q.s |
| Extruded colloidal oatmeal | 5 |

### Method

### Stage 1

The citric acid, sodium citrate and hydroxyethylcellulose are added to the water. Using a propellor stirrer, the mixture is stirred until dispersed. The xanthan gum is pre-dispersed in the glycerin and this is then added to the bulk, which is then heated to 70°C.

### Stage 2

The isopropyl palmitate, arachidyl propionate, dimethicone, steareth-21, steareth-2, cetyl alcohol, tribehenin , glyceryl stearate, paraffinum liquidum are mixed and heated to 70°C to melt the waxes.

### Stage 3

Using a homogeniser, stage 2 is added to stage 1 and is mixed until emulsified and uniform. The emulsion is cooled to below 35° C using stirring. Once below 35°C, the remaining materials are added, including the Extruded colloidal oatmeal. The product is made to weight using purified water, and mixed until uniform.

### Example 14 Aftersun Lotion (Formulation 6)

| | %w/w |
|---|---|
| Aqua | to 100 |
| Hydrated silica | 5 |
| Isopropyl palmitate | 4 |
| Arachidyl propionate | 2 |
| Dimethicone | 2 |
| Glycerin | 2 |
| Steareth-21 | 1.96 |
| Steareth-2 | 1.683 |
| Cetyl alcohol | 1 |
| Tribehenin | 1 |
| Glyceryl stearate | 1 |
| Paraffinum liquidum | 0.994 |
| Panthenol | 0.75 |
| Parfum | 0.3 |
| Xanthan gum | 0.3 |
| Sodium citrate | 0.25 |
| Tocopheryl acetate | 0.2 |
| Hydroxyethylcellulose | 0.1 |
| Bisabolol | 0.095 |
| Citric acid | 0.05 |
| Preservative | q.s |
| Extruded colloidal oatmeal | 10 |

### Method

### Stage 1

The citric acid, sodium citrate and hydroxyethylcellulose are added to the water. Using a propellor stirrer, the mixture is stirred until dispersed. The xanthan gum is pre-dispersed in the glycerin and this is then added to the bulk, which is then heated to 70°C.

### Stage 2

The isopropyl palmitate, arachidyl propionate, dimethicone, steareth-21, steareth-2, cetyl alcohol, tribehenin , glyceryl stearate, paraffinum liquidum are mixed and heated to 70°C to melt the waxes.

### Stage 3

Using a homogeniser, stage 2 is added to stage 1 and is mixed until emulsified and uniform. The emulsion is cooled to below 35° C using stirring. Once below 35°C, the remaining materials are added, including the Extruded colloidal oatmeal. The product is made to weight using purified water, and mixed until uniform.

### Example 15 Day Cream (Formulation 7)

| | %w/w |
|---|---|
| Aqua | to 100 |
| Butylene glycol | 5 |
| Dicaprylyl maleate | 4 |
| Paraffinum liquidum | 4 |
| Octyl methoxycinnamate | 3 |
| Petrolatum | 3 |
| Cetyl Alcohol | 2 |
| Glycerin | 2 |
| Dimethicone | 2 |
| Cetearyl alcohol | 1.6 |
| Butyl methoxydibenzoylmethane | 1 |
| Hydroxyethylcellulose | 0.4 |
| PEG-20 stearate | 0.4 |
| Polyacrylamide | 0.4 |
| Parfum | 0.3 |
| C13-14 isoparaffin | 0.215 |
| Retinyl palmitate | 0.15 |
| Tetrasodium EDTA | 0.1 |
| Citric acid | 0.08 |
| Laureth-7 | 0.055 |
| BHT | 0.0024 |
| Extruded colloidal oatmeal | 1 |
| Preservative | q.s |

### Method

### Stage 1

Tetrasodium EDTA and citric acid are added to the water using a propellor stirrer. The hydroxyethylcellulose is added and dispersed using a homogeniser. butylene glycol, glycerin and methylparaben are added and the bulk is heated to 70°C.

### Stage 2

The dicaprylyl maleate, paraffinum liquidum, octyl methoxycinnamate, petrolatum, cetyl alcohol, dimethicone, cetearyl alcohol, butyl methoxydibenzoylmethane, PEG-20 stearate, C13-14 isoparaffin, laureth-7 and BHT are mixed and heated to 70°C to melt the waxes.

### Stage 3

Using a homogeniser, stage 2 is added to stage 1 and the bulk is mixed until emulsified and stable. The product is then cooled to below 35°C using stirring. The remaining raw materials, including the Extruded colloidal oatmeal are added and the product is mixed using a propellor stirrer until uniform. The product is made to weight using purified water.

### Example 16 Day Cream (Formulation 8)

| | %w/w |
|---|---|
| Aqua | to 100 |
| Butylene glycol | 5 |
| Dicaprylyl maleate | 4 |
| Paraffinum liquidum | 4 |
| Octyl methoxycinnamate | 3 |
| Petrolatum | 3 |
| Cetyl Alcohol | 2 |
| Glycerin | 2 |
| Dimethicone | 2 |
| Cetearyl alcohol | 1.6 |
| Butyl methoxydibenzoylmethane | 1 |
| Hydroxyethylcellulose | 0.4 |
| PEG-20 stearate | 0.4 |
| Polyacrylamide | 0.4 |
| Parfum | 0.3 |
| C13-14 isoparaffin | 0.215 |
| Retinyl palmitate | 0.1782 |
| Tetrasodium EDTA | 0.1 |
| Citric acid | 0.08 |
| Laureth-7 | 0.055 |
| BHT | 0.0024 |
| Extruded colloidal oatmeal | 5 |
| Preservative | q.s |

### Method

### Stage 1

Tetrasodium EDTA and citric acid are added to the water using a propellor stirrer. The hydroxyethylcellulose is added and dispersed using a homogeniser. butylene glycol, glycerin and methylparaben are added and the bulk is heated to 70°C.

### Stage 2

The dicaprylyl maleate, paraffinum liquidum, octyl methoxycinnamate, petrolatum, cetyl alcohol, dimethicone, cetearyl alcohol, butyl methoxydibenzoylmethane, PEG-20 stearate, C13-14 isoparaffin, laureth-7 and BHT are mixed and heated to 70°C to melt the waxes.

### Stage 3

Using a homogeniser, stage 2 is added to stage 1 and the bulk is mixed until emulsified and stable. The product is then cooled to below 35°C using stirring. The remaining raw materials, including the Extruded colloidal oatmeal are added and the product is mixed using a propellor stirrer until uniform. The product is made to weight using purified water.

### Example 17 Sun Lotion (Formulation 9)

| | %w/w |
|---|---|
| Aqua | to 100 |
| C12-15 Alkyl Benzoate | 8 |
| Butylene glycol | 5 |
| Butyl methoxydibenzoylmethane | 2.2 |
| Dimethicone | 2 |
| Polyglyceryl-3 methylglucose distearate | 2 |
| PVP/hexadecene copolymer | 1.75 |
| Octyl methoxycinnamate | 1.7 |
| Theobroma cacao | 0.5 |
| Parfum | 0.5 |
| Tocopheryl acetate | 0.2 |
| Acrylates/vinyl isodecanoate crosspolymer | 0.15 |
| Potassium hydroxide | 0.034 |
| Tetrasodium EDTA | 0.02 |
| Preservative | q.s |
| Extruded colloidal oatmeal | 3 |

### Method

### Stage 1

The EDTA is dispersed into the water. Using a propellor stirrer, the acrylates/vinyl isodecanoate crosspolymer are added and dispersed and hydrated. Butylene glycol is added and the aqueous phase is heated to 70°C.

### Stage 2

The C12-15 alkyl benzoate, butyl methoxydibenzoylmethane, dimethicone, polyglyceryl-3 methylglucose distearate, PVP/hexadecene copolymer, octyl methoxycinnamate, theobroma cacao and tocopheryl acetate are mixed and heated to 70°C to melt the waxes.

### Stage 3

Using a homogeniser, stage 2 is added to stage 1 and the bulk is mixed until emulsified and uniform. The emulsion is cooled to below 35°C with stirring. The remaining materials, including the Extruded colloidal oatmeal are added and mixed. The product is made to weight using purified water and stirred until uniform.

### Example 18 Sun Lotion (Formulation 10)

| | %w/w |
|---|---|
| Aqua | to 100 |
| C12-15 Alkyl Benzoate | 8 |
| Butylene glycol | 5 |
| Butyl methoxydibenzoylmethane | 2.2 |
| Dimethicone | 2 |
| Polyglyceryl-3 methylglucose distearate | 2 |
| PVP/hexadecene copolymer | 1.75 |
| Octyl methoxycinnamate | 1.7 |
| Theobroma cacao | 0.5 |
| Parfum | 0.5 |
| Tocopheryl acetate | 0.2 |
| Acrylates/vinyl isodecanoate crosspolymer | 0.15 |
| Potassium hydroxide | 0.034 |
| Tetrasodium EDTA | 0.02 |
| Preservative | q.s |
| Extruded colloidal oatmeal | 5 |

### Method

### Stage 1

The EDTA is dispersed into the water. Using a propellor stirrer, the acrylates/vinyl isodecanoate crosspolymer are added and dispersed and hydrated. Butylene glycol is added and the aqueous phase is heated to 70°C.

### Stage 2

The C12-15 alkyl benzoate, butyl methoxydibenzoylmethane, dimethicone, polyglyceryl-3 methylglucose distearate, PVP/hexadecene copolymer, octyl methoxycinnamate, theobroma cacao and tocopheryl acetate are mixed and heated to 70°C to melt the waxes.

### Stage 3

Using a homogeniser, stage 2 is added to stage 1 and the bulk is mixed until emulsified and uniform. The emulsion is cooled to below 35°C with stirring. The remaining materials, including the Extruded colloidal oatmeal are added and mixed. The product is made to weight using purified water and stirred until uniform.

### Example 19 Sunburn Treatment (Formulation 11)

| | %w/w |
|---|---|
| Aqua | to 100 |
| Petrolatum | 3 |
| Cetyl Alcohol | 2 |
| Dimethicone | 2 |
| Glycerin | 2 |
| Ceteath-20 | 1.7 |
| Paraffinum Liquidum | 1 |
| Sodium chloride | 0.8 |
| Theobroma cacao | 0.7 |
| Glyceryl stearate | 0.5 |
| Parfum | 0.3 |
| Allantoin | 0.2 |
| Hydroxyethylcellulose | 0.1 |
| Triclosan | 0.1 |
| Citric acid | 0.02 |
| Preservative | q.s |
| Extruded colloidal oatmeal | 10 |

### Method

### Stage 1

Into the water, sodium chloride and citric acid are added and dispersed. Using a propellor stirrer, hydroxyethylcellulose is added and dispersed. This phase is then heated to 70°C.

### Stage 2

The petrolatum, cetyl alcohol, dimethicone, ceteath-20, paraffinum liquidum, theobroma cacao and glyceryl stearate are mixed and heated to 70°C to melt the waxes.

### Stage 3

Using a homogeniser, stage 2 is added to stage 1, this is mixed until emulsified and uniform. The emulsion is then cooled to below 35°C with stirring. The remaining materials, including the Extruded colloidal oatmeal are then added and mixed. The product is made to weight using purified water and stirred until uniform.

### Example 20 Sunburn Treatment (Formulation 12)

| | %w/w |
|---|---|
| Aqua | to 100 |
| Petrolatum | 3 |
| Cetyl Alcohol | 2 |
| Dimethicone | 2 |
| Glycerin | 2 |
| Ceteath-20 | 1.7 |
| Paraffinum Liquidum | 1 |
| Sodium chloride | 0.8 |
| Theobroma cacao | 0.7 |
| Glyceryl stearate | 0.5 |
| Parfum | 0.3 |
| Allantoin | 0.2 |
| Hydroxyethylcellulose | 0.1 |
| Triclosan | 0.1 |
| Citric acid | 0.02 |
| Preservative | q.s |
| Extruded colloidal oatmeal | 7 |

### Method

### Stage 1

Into the water, sodium chloride and citric acid are added and dispersed. Using a propellor stirrer, hydroxyethylcellulose is added and dispersed. This phase is then heated to 70°C.

### Stage 2

The petrolatum, cetyl alcohol, dimethicone, ceteath-20, paraffinum liquidum, theobroma cacao and glyceryl stearate are mixed and heated to 70°C to melt the waxes.

### Stage 3

Using a homogeniser, stage 2 is added to stage 1, this is mixed until emulsified and uniform. The emulsion is then cooled to below 35°C with stirring. The remaining materials, including the Extruded colloidal oatmeal are then added and mixed. The product is made to weight using purified water and stirred until uniform.

### Example 21 Eye Cream (Formulation 13)

| | %w/w |
|---|---|
| Aqua | to 100 |
| Butylene glycol | 6 |
| Paraffinum liquidum | 5 |
| Octyl methoxycinnamate | 4 |
| Dimethicone | 2 |
| Petrolutum | 2 |
| Cetearyl octanoate | 1.8 |
| Cetearyl alcohol | 1.6 |
| Glyceryl stearate | 1.5 |
| Cetyl alcohol | 1 |
| Prunus dulcis | 1 |
| Glycerin | 0.57 |
| Hydrogenated vegetable glycerides citrate | 0.5 |
| Tocopheryl acetate | 0.5 |
| Bisabolol | 0.475 |
| Panthenol | 0.45 |
| Sodium phosphate | 0.42 |
| PEG-20 stearate | 0.4 |
| Isopropyl myristate | 0.2 |
| Carbomer | 0.15 |
| PEG-12 isostearate | 0.125 |
| Allantoin | 0.1 |
| Tetrasodium EDTA | 0.1 |
| Lactic acid | 0.088 |
| Disodium phophate | 0.083 |
| Potassium hydroxide | 0.051 |
| Extruded colloidal oatmeal | 1 |
| Preservative | q.s |

### Method

### Stage 1

Into the water, citric acid, EDTA, sodium phosphate, disodium phosphate and lactic acid are added and dispersed. Using a homogeniser, carbomer is added and hydrated. The aqueous phase is then heated to 70°C.

### Stage 2

The paraffinum liquidum, octyl methoxycinnamate, dimethicone, petrolatum, cetearyl octanoate, cetearyl alcohol, glyceryl stearate, cetyl alcohol, hydrogenated vegetable glycerides citrate, tocopheryl acetate, PEG-20 stearate, isopropyl myristate and PEG-12 isostearate are mixed and heated to 70°C to melt the waxes.

### Stage 3

Using a homogeniser, stage 2 is added to stage 1 and this is mixed until emulsified and uniform. The emulsion is then cooled to below 35°C using stirring. The remaining materials, including the Extruded colloidal oatmeal are then added and mixed. The product is then made to weight using purified water and is stirred until uniform.

### Example 22 Eye Cream (Formulation 14)

| | %w/w |
|---|---|
| Aqua | to 100 |
| Butylene glycol | 6 |
| Paraffinum liquidum | 5 |
| Octyl methoxycinnamate | 4 |
| Dimethicone | 2 |
| Petrolutum | 2 |
| Cetearyl octanoate | 1.8 |
| Cetearyl alcohol | 1.6 |
| Glyceryl stearate | 1.5 |
| Cetyl alcohol | 1 |
| Prunus dulcis | 1 |
| Glycerin | 0.57 |
| Hydrogenated vegetable glycerides citrate | 0.5 |
| Tocopheryl acetate | 0.5 |
| Bisabolol | 0.475 |
| Panthenol | 0.45 |
| Sodium phosphate | 0.42 |
| PEG-20 stearate | 0.4 |
| Isopropyl myristate | 0.2 |
| Carbomer | 0.15 |
| PEG-12 isostearate | 0.125 |
| Allantoin | 0.1 |
| Tetrasodium EDTA | 0.1 |
| Lactic acid | 0.088 |
| Disodium phophate | 0.083 |
| Potassium hydroxide | 0.051 |
| Extruded colloidal oatmeal | 4 |
| Preservative | q.s |

### Method

### Stage 1

Into the water, citric acid, EDTA, sodium phosphate, disodium phosphate and lactic acid are added and dispersed. Using a homogeniser, carbomer is added and hydrated. The aqueous phase is then heated to 70°C.

### Stage 2

The paraffinum liquidum, octyl methoxycinnamate, dimethicone, petrolatum, cetearyl octanoate, cetearyl alcohol, glyceryl stearate, cetyl alcohol, hydrogenated vegetable glycerides citrate, tocopheryl acetate, PEG-20 stearate, isopropyl myristate and PEG-12 isostearate are mixed and heated to 70°C to melt the waxes.

### Stage 3

Using a homogeniser, stage 2 is added to stage 1 and this is mixed until emulsified and uniform. The emulsion is then cooled to below 35°C using stirring. The remaining materials, including the Extruded colloidal oatmeal are then added and mixed. The product is then made to weight using purified water and is stirred until uniform.

### Example 23 Eye Gel (Formulation 15)

| | %W/W |
|---|---|
| Aqua | to 100 |
| PVP/VA copolymer | 2 |
| Propylene glycol | 2 |
| Carbomer | 1 |
| PEG-40 hydrogenated castor oil | 1 |
| Panthenol | 1 |
| Sodium hydroxide | 0.3 |
| Phenoxyethanol | 0.2 |
| Tetrasodium EDTA | 0.1 |
| Extruded colloidal oatmeal | 0.5 |

### Method

### Stage 1

The EDTA, Methyldibromo glutaronitrile, PVP/VA copolymer and Carbomer were added to the water and mixed using a homogeniser to ensure that the polymers were hydrated.

### Stage 2

With continued homogenising, the Cystine hydroxypropyl polysiloxane was added and mixed into the product.

### Stage 3

The remaining materials, including the Extruded colloidal oatmeal were added individually and mixed using a prop. Strirrer until the product was homogenous.

### Example 24 Refreshing Cream (Formulation 16)

| | %w/w |
|---|---|
| Aqua | to 100 |
| Butylene glycol | 7.5 |
| Silica | 7.2 |
| Arabinogalactan | 5.35 |
| Dimethicone | 5.35 |
| Petrolatum | 5.35 |
| Hydrated silica | 3.75 |
| Steareth-2 | 2.7 |
| Prunus dulcis | 2.7 |
| Steareth-21 | 0.9 |
| PVP/hexadecene copolymer | 0.8 |
| Carbomer | 0.32 |
| Sodium PCA | 0.2 |
| Parfum | 0.2 |
| Hydroxyethylcellulose | 0.16 |
| Potassium hydroxide | 0.1 |
| Propylene glycol | 0.1 |
| Extruded colloidal oatmeal | 1 |
| Preservative | q.s |

### Method

### Stage 1

Into the water, the carbomer is added and hydrated using a homogeniser. The aqueous phase is then heated to 70°C.

### Stage 2

The silica, arabinogalactan, PVP/hexadecene copolymer, dimethicone, petrolatum, hydrated silica, steareth-2 and steareth-21 are mixed and heated to 70°C to melt the waxes.

### Stage 3

Using a homogeniser, stage 2 is added to stage 1 and this is mixed until emulsified and uniform. The emulsion is then cooled to below 35°C using stirring. The remaining materials, including the Extruded colloidal oatmeal are then added and mixed. The product is then made to weight using purified water and is stirred until uniform.

### Example 25 Skin Protection Lotion (Formulation 17)

| | %w/w |
|---|---|
| Aqua | to 100 |
| Dimethicone | 5 |
| Glycerin | 3 |
| Kaolin | 3 |
| Dicaprylyl maleate | 2.5 |
| Isopropyl myristate | 2.5 |
| Stearate-2 | 2 |
| Octyl methoxycinnamate | 1 |
| Steareth-21 | 1 |
| Cetyl alcohol | 0.75 |
| Butyl methoxydibenzoylmethane | 0.5 |
| Propylene glycol | 0.5 |
| Hydroxyethylcellulose | 0.4 |
| Xanthan gum | 0.24 |
| Serica | 0.1 |
| Sodium C8-16 isoalkylsuccinyl lactoglobulin sulfonate | 0.1 |
| Tetrasodium EDTA | 0.1 |
| Citric acid | 0.05 |
| Extruded colloidal oatmeal | 1 |
| Preservative | q.s |

### Method

### Stage 1

Into the water, the citric acid and EDTA are added and dispersed. The hydroxyethylcellulose is added and hydrated using a propellor stirrer. Xanthan gum is pre-dispersed in glycerin and added to the bulk. This is stirred until uniform. The aqueous phase is then heated to 70°C.

### Stage 2

The dimethicone, dicaprylyl maleate, isopropyl myristate, stearate-2, octyl methoxycinnamate, steareth-21, cetyl alcohol and butyl methoxydibenzoylmethane are mixed and heated to 70 °C to melt the waxes.

### Stage 3

Using a homogeniser, stage 2 is added to stage 1 and this is mixed until emulsified and uniform. The emulsion is then cooled to below 35°C using stirring. The remaining materials, including the Extruded colloidal oatmeal are then added and mixed. The product is then made to weight using purified water and is stirred until uniform.

### Example 26 Night Cream (Formulation 18)

| | |
|---|---|
| | %w/w |
| Aqua | to 100 |
| Glycerin | 5 |
| Paraffinum liquidum | 4.5 |
| Dicaprylyl maleate | 3 |
| Dimethicone | 3 |
| Petrolatum | 3 |
| Paraffin | 2.9 |
| Cetyl alcohol | 2 |
| Steareth-2 | 2 |
| Glyceryl stearate | 1.5 |
| Butyrospermum parkii | 1.5 |
| Steareth-21 | 1 |
| Mannitol | 1 |
| Cera microcristallina | 0.262 |
| Buxus chinensis | 0.5 |
| Propylene glycol | 0.48 |
| Parfum | 0.4 |
| Borago officinalis | 0.3 |
| Hydroxyethylcellulose | 0.3 |
| Lactis proteinum | 0.3 |
| Xanthan gum | 0.25 |
| Alcohol denat. | 0.08 |
| Sodium citrate | 0.08 |
| Lecithin | 0.075 |
| BHT | 0.05 |
| Faex | 0.04 |
| Phospholipids | 0.03 |
| Citric acid | 0.025 |
| Extruded colloidal oatmeal | 5 |
| Preservative | q.s |

### Method

### Stage 1

Into the water, the citric acid and sodium citrate are added and dispersed. The hydroxyethylcellulose is added and hydrated using a propellor stirrer. Xanthan gum is pre-dispersed in glycerin and added to the bulk. This is stirred until uniform. The aqueous phase is then heated to 70°C.

### Stage 2

The paraffinum liquidum, dicaprylyl maleate, dimethicone, petrolatum, paraffin, cetyl alcohol, steareth-2, glyceryl stearate, steareth-21, cera microcristallina and BHT are mixed and heated to 70°C to melt the waxes.

### Stage 3

Using a homogeniser, stage 2 is added to stage 1 and this is mixed until emulsified and uniform. The emulsion is then cooled to below 35°C using stirring. The remaining materials, including the Extruded colloidal oatmeal are then added and mixed. The product is then made to weight using purified water and is stirred until uniform.

### Example 27 Sun Lotion for Sensitive Skin (Formulation 19)

| | %w/w |
|---|---|
| Aqua | to 100 |
| C12-15 alkyl benzoate | 12 |
| Butylene glycol | 5 |
| Octyl methoxycinnamate | 3.8 |
| Butyl methoxydibenzoylmethane | 3 |
| Dimethicone | 2 |
| Polyglyceryl-3 methylglucose distearate | 2 |
| PVP/hexadecene copolymer | 1.75 |
| C18-36 acid glycol ester | 1.5 |
| Polysorbate 60 | 0.5 |
| Titanium dioxide | 0.3 |
| Tocopheryl acetate | 0.2 |
| Acrylates/vinyl isodecanoate crosspolymer | 0.14 |
| Potassium hydroxide | 0.035 |
| Tetrasodium EDTA | 0.02 |
| Preservative | q.s |
| Extruded colloidal oatmeal | 5 |

### Method

### Stage 1

Into the water, citric acid is added and dispersed. The acrlyates/vinyl isodecanoate crosspolymer are added and dispersed using a propellor stirrer. The aqueous phase is then heated to 70°C.

### Stage 2

The C12-15 alkyl benzoate, PVP/hexadecene copolymer, octyl methoxycinnamate, butyl methoxydibenzoylmethane, dimethicone, polyglyceryl-3 methylglucose distearate, C18-36 acid glycol ester, polysorbate 60, titanium dioxide and tocopheryl acetate are mixed and heated to 70°C to melt the waxes.

### Stage 3

Using a homogeniser, stage 2 is added to stage 1 and this is mixed until emulsified and uniform. The emulsion is then cooled to below 35°C using stirring. The remaining materials, including the Extruded colloidal oatmeal are then added and mixed. The product is then made to weight using purified water and is stirred until uniform.

### Example 28 Sun Lotion for Sensitive Skin (Formulation 20)

| | %w/w |
|---|---|
| Aqua | to 100 |
| C12-15 alkyl benzoate | 12 |
| Butylene glycol | 5 |
| Octyl methoxycinnamate | 3.8 |
| Butyl methoxydibenzoylmethane | 3 |
| Dimethicone | 2 |
| Polyglyceryl-3 methylglucose distearate | 2 |
| PVP/hexadecene copolymer | 1.75 |
| C18-36 acid glycol ester | 1.5 |
| Polysorbate 60 | 0.5 |
| Titanium dioxide | 0.3 |
| Tocopheryl acetate | 0.2 |
| Acrylates/vinyl isodecanoate crosspolymer | 0.14 |
| Potassium hydroxide | 0.035 |
| Tetrasodium EDTA | 0.02 |
| Preservative | q.s |
| Extruded colloidal oatmeal | 10 |

### Method

### Stage 1

Into the water, citric acid is added and dispersed. The acrylates/vinyl isodecanoate crosspolymer are added and dispersed using a propellor stirrer. The aqueous phase is then heated to 70°C.

### Stage 2

The C12-15 alkyl benzoate, PVP/hexadecene copolymer, octyl methoxycinnamate, butyl methoxydibenzoylmethane, dimethicone, polyglyceryl-3 methylglucose distearate, C18-36 acid glycol ester, polysorbate 60, titanium dioxide and tocopheryl acetate are heated to 70°C to melt the waxes.

### Stage 3

Using a homogeniser, stage 2 is added to stage 1 and this is mixed until emulsified and uniform. The emulsion is then cooled to below 35°C using stirring. The remaining materials, including the Extruded colloidal oatmeal are then added and mixed. The product is then made to weight using purified water and is stirred until uniform.

### Example 29 Sun Cream for Sensitive Skin (Formulation 21)

| | %w/w |
|---|---|
| Aqua | to 100 |
| Octyl stearate | 13.5 |
| Zinc oxide | 13.5 |
| Isopropyl myristate | 5 |
| Butylene glycol | 3 |
| Isohexadecane | 3 |
| Titanium dioxide | 2 |
| Polyglyceryl-3 oleate | 1.75 |
| Cetyl dimethicone copolyol | 1.35 |
| Magnesium sulfate | 0.75 |
| Sodium chloride | 0.75 |
| Aluminium stearate | 0.18 |
| Alumina | 0.15 |
| Lecithin | 0.13 |
| Isopropyl palmitate | 0.05 |
| Extruded colloidal oatmeal | 1 |

### Method

### Stage 1

Into the water, magnesium sulfate, sodium chloride and butylene glycol are added and dispersed. The aqueous phase is then heated to 70°C.

### Stage 2

The octyl stearate, isopropyl myristate, isohexadecane, titanium dioxide, polyglyceryl-3 oleate, cetyl dimethicone copolyol, aluminium stearate, lecithin and isopropyl palmitate are mixed and heated to 70°C to melt the waxes.

### Stage 3

Using a propellor stirrer, stage 2 is added to stage 1. Once uniform, the emulsion is transferred to a homogeniser and mixed to generate the viscosity. The emulsion is then cooled to below 35°C using stirring. The remaining materials, including the Extruded colloidal oatmeal are then added and mixed. The product is then made to weight using purified water and is stirred until uniform.

### Example 30 Sun Cream for Sensitive Skin (Formulation 22)

| | %w/w |
|---|---|
| Aqua | to 100 |
| Octyl stearate | 13.5 |
| Zinc oxide | 13.5 |
| Isopropyl myristate | 5 |
| Butylene glycol | 3 |
| Isohexadecane | 3 |
| Titanium dioxide | 2 |
| Polyglyceryl-3 oleate | 1.75 |
| Cetyl dimethicone copolyol | 1.35 |
| Magnesium sulfate | 0.75 |
| Sodium chloride | 0.75 |
| Aluminium stearate | 0.18 |
| Alumina | 0.15 |
| Lecithin | 0.13 |
| Isopropyl palmitate | 0.05 |
| Extruded colloidal oatmeal | 10 |

### Method

### Stage 1

Into the water, magnesium sulfate, sodium chloride and butylene glycol are added and dispersed. The aqueous phase is then heated to 70°C.

### Stage 2

The octyl stearate, isopropyl myristate, isohexadecane, titanium dioxide, polyglyceryl-3 oleate, cetyl dimethicone copolyol, aluminium stearate, lecithin and isopropyl palmitate are mixed and heated to 70°C to melt the waxes.

### Stage 3

Using a propellor stirrer, stage 2 is added to stage 1. Once uniform, the emulsion is transferred to a homogeniser and mixed to generate the viscosity. The emulsion is then cooled to below 35°C using stirring. The remaining materials, including the Extruded colloidal oatmeal are then added and mixed. The product is then made to weight using purified water and is stirred until uniform.

### Example 31 Anti-ageing Foundation (Formulation 23)

| | %w/w |
|---|---|
| Aqua | to 100 |
| Butylene glycol | 9.8 |
| Cetearyl isononanoate | 4.9 |
| Dimethicone | 3.2 |
| Glycerin | 1.96 |
| Silica | 1.9 |
| Caprylic/capric triglyceride | 1.67 |
| Paraffinum liquidum | 1.67 |
| Petrolatum | 1.67 |
| Hydrogenated coco-glycerides | 1.67 |
| Cetearyl octanoate | 1.5 |
| Cetearyl alcohol | 1.35 |
| Octyl methoxycinnamate | 1.28 |
| Talc | 1 |
| Glyceryl stearate | 0.95 |
| PEG-100 stearate | 0.9 |
| Butyl methoxydibenzoylmethane | 0.6 |
| Saccharide isomerate | 0.54 |
| Lactic acid | 0.45 |
| Sodium polyacrylate | 0.45 |
| Boron nitride | 0.42 |
| Sodium PCA | 0.4 |
| Borago officinalis | 0.4 |
| Tocopheryl acetate | 0.4 |
| PVP/hexadecene copolymer | 0.4 |
| PEG-20 stearate | 0.33 |
| Glycolic acid | 0.2 |
| Sodium stearoyl lactylate | 0.2 |
| Isopropyl myristate | 0.17 |
| Polyaminopropyl biguanide | 0.16 |
| Tetrasodium EDTA | 0.1 |
| Xanthan gum | 0.1 |
| Citric acid | 0.06 |
| Alcohol denat. | 0.04 |
| Lecithin | 0.037 |
| Preservative | q.s |
| Extruded colloidal oatmeal | 1 |

### Method

### Stage 1

Into the water, citric acid, EDTA and lactic acid are added and dispersed. Xanthan gum is pre-dispersed in butylene glycol and is added to the bulk. The aqueous phase is then heated to 70°C.

### Stage 2

The cetearyl isononanoate, dimethicone, silica, PVP/hexadecene copolymer, caprylic/capric triglyceride, paraffinum liquidum, petrolatum, hydrogenated coco-glycerides, cetearyl octanoate, cetearyl alcohol, octyl methoxycinnamate, talc, glyceryl stearate, PEG-100 stearate, butyl methoxydibenzoylmethane, borago officinalis, tocopheryl acetate, sodium stearoyl lactylate, isopropyl myristate and lecithinoil phase are mixed and heated to 70°C to melt the waxes.

### Stage 3

Using a homogeniser, stage 2 is added to stage 1 and this is mixed until emulsified and uniform. The emulsion is then cooled to below 35°C using stirring. The remaining materials, including the Extruded colloidal oatmeal are then added and mixed. The product is then made to weight using purified water and is stirred until uniform.

### Example 32 Anti-Ageing Foundation (Formulation 24)

| | %w/w |
|---|---|
| Aqua | to 100 |
| Butylene glycol | 9.8 |
| Cetearyl isononanoate | 4.9 |
| Dimethicone | 3.2 |
| Glycerin | 1.96 |
| Silica | 1.9 |
| Caprylic/capric triglyceride | 1.67 |
| Paraffinum liquidum | 1.67 |
| Petrolatum | 1.67 |
| Hydrogenated coco-glycerides | 1.67 |
| Cetearyl octanoate | 1.5 |
| Cetearyl alcohol | 1.35 |
| Octyl methoxycinnamate | 1.28 |
| Talc | 1 |
| Glyceryl stearate | 0.95 |
| PEG-100 stearate | 0.9 |
| Butyl methoxydibenzoylmethane | 0.6 |
| Saccharide isomerate | 0.54 |
| Lactic acid | 0.45 |
| Sodium polyacrylate | 0.45 |
| Boron nitride | 0.42 |
| Iron Oxide | 3 |
| Titanium Dioxide | 1 |
| Sodium PCA | 0.4 |
| Borago officinalis | 0.4 |
| Tocopheryl acetate | 0.4 |
| PVP/hexadecene copolymer | 0.4 |
| PEG-20 stearate | 0.33 |
| Glycolic acid | 0.2 |
| Sodium stearoyl lactylate | 0.2 |
| Isopropyl myristate | 0.17 |
| Polyaminopropyl biguanide | 0.16 |
| Tetrasodium EDTA | 0.1 |
| Xanthan gum | 0.1 |
| Citric acid | 0.06 |
| Alcohol denat. | 0.04 |
| Lecithin | 0.037 |
| Preservative | q.s |
| Extruded colloidal oatmeal | 10 |

### Method

### Stage 1

Into the water, citric acid, EDTA and Lactic acid are added and dispersed. Xanthan gum is pre-dispersed in butylene glycol and is added to the bulk. The aqueous phase is then heated to 70°C.

### Stage 2

The cetearyl isononanoate, dimethicone, Silica, PVP/hexadecene copolymer, caprylic/capric triglyceride, paraffinum liquidum, petrolatum, hydrogenated coco-glycerides, cetearyl octanoate, cetearyl alcohol, octyl methoxycinnamate, talc, glyceryl stearate, PEG-100 stearate, butyl methoxydibenzoylmethane, borago officinalis, tocopheryl acetate, sodium stearoyl lactylate, isopropyl myristate and
lecithinoil phase are mixed and heated to 70°C to melt the waxes.

### Stage 3

Using a homogeniser, stage 2 is added to stage 1 and this is mixed until emulsified and uniform. The emulsion is then cooled to below 35°C using stirring. The remaining materials, including the Extruded colloidal oatmeal are then added and mixed. The product is then made to weight using purified water and is stirred until uniform.

### Example 33 Sun Spray - SPF15 (Formulation 25)

| | %w/w |
|---|---|
| Aqua | to 100 |
| Dicaprylyl maleate | 12 |
| Butylene glycol | 5 |
| Octyl methoxycinnamate | 4 |
| Butyl methoxydibenzoylmethane | 3.5 |
| Dimethicone | 3 |
| Polyglyceryl-3 methylglucose distearate | 3 |
| Acrylates/octylacrylamide copolymer | 2 |
| C18-36 acid glycol ester | 1.5 |
| Triethanolamine | 0.5 |
| Tocopheryl acetate | 0.2 |
| Acrylates/vinyl isodecanoate crosspolymer | 0.05 |
| Tetrasodium EDTA | 0.02 |
| Potassium hydroxide | 0.015 |
| Preservative | q.s |
| Extruded colloidal oatmeal | 1 |

### Method

### Stage 1

Into the water, EDTA is added and dispersed. Acrylates/vinyl isodecanoate crosspolymer are added and dispersed using a propellor stirrer. Butylene glycol is added and dispersed. The aqueous phase is then heated to 70°C.

### Stage 2

The dicaprylyl maleate, Acrylates/octylacrylamide copolymer, octyl methoxycinnamate, butyl methoxydibenzoylmethane, dimethicone, polyglyceryl-3 methylglucose, C18-36 acid glycol ester and tocopheryl acetate are mixed and heated to 80°C to melt the waxes.

### Stage 3

Using a homogeniser, stage 2 is added to stage 1 and this is mixed until emulsified and uniform. The emulsion is then cooled to below 35°C using stirring. The remaining materials, including the Extruded colloidal oatmeal are then added and mixed. The product is then made to weight using purified water and is stirred until uniform.

### Example 34 Sun Spray (Formulation 26)

| | %w/w |
|---|---|
| Aqua | to 100 |
| Dicaprylyl maleate | 12 |
| Butylene glycol | 5 |
| Octyl methoxycinnamate | 4 |
| Butyl methoxydibenzoylmethane | 3.5 |
| Dimethicone | 3 |
| Polyglyceryl-3 methylglucose distearate | 3 |
| Acrylates/octylacrylamide copolymer | 2 |
| C18-36 acid glycol ester | 1.5 |
| Triethanolamine | 0.5 |
| Tocopheryl acetate | 0.2 |
| Acrylates/vinyl isodecanoate crosspolymer | 0.05 |
| Tetrasodium EDTA | 0.02 |
| Potassium hydroxide | 0.015 |
| Preservative | q.s |
| Extruded colloidal oatmeal | 0.5 |

### Method

### Stage 1

Into the water, EDTA is added and dispersed. Acrylates/vinyl isodecanoate crosspolymer are added and dispersed using a propellor stirrer. Butylene glycol is added and dispersed. The aqueous phase is then heated to 70°C.

### Stage 2

The dicaprylyl maleate, Acrylates/octylacrylamide copolymer, octyl methoxycinnamate, butyl methoxydibenzoylmethane, dimethicone, polyglyceryl-3 methylglucose, C18-36 acid glycol ester and tocopheryl acetate are mixed and heated to 70°C to melt the waxes.

### Stage 3

Using a homogeniser, stage 2 is added to stage 1 and this is mixed until emulsified and uniform. The emulsion is then cooled to below 35°C using stirring. The remaining materials, including the Extruded colloidal oatmeal are then added and mixed. The product is then made to weight using purified water and is stirred until uniform.

### Example 35 Toner & Cleanser 2 In 1 (Formulation 27)

| | %w/w |
|---|---|
| Alcohol denat. | 48 |
| Aqua | to 100 |
| PEG-8 | 6 |
| Glycerin | 2 |
| Propylene glycol | 0.5 |
| Sodium C8-16 isoalkylsuccinyl lactoglobulin sulfonate | 0.02 |
| Laminaria saccharina | 0.01 |
| Hamamelis virginiana | 0.006 |
| Citrullus vulgaris | 0.001 |
| Preservative | q.s |
| Extruded colloidal oatmeal | 2 |

### Method

### Stage 1

Into the water, alcohol denat. Is added and dispersed until uniform. Using a propellor stirrer, all materials including the Extruded colloidal oatmeal, are slowly added and stirred until uniform. The product is made to weight using purified water and stirred until uniform.

### Example 36 Toner & Cleanser 2 In 1 (Formulation 28)

| | %w/w |
|---|---|
| Alcohol denat. | 48 |
| Aqua | to 100 |
| PEG-8 | 6 |
| Glycerin | 2 |
| Propylene glycol | 0.5 |
| Sodium C8-16 isoalkylsuccinyl lactoglobulin sulfonate | 0.02 |
| Laminaria saccharina | 0.01 |
| Hamamelis virginiana | 0.006 |
| Citrullus vulgaris | 0.001 |
| Preservative | q.s |
| Extruded colloidal oatmeal | 5 |

### Method

### Stage 1

Into the water, alcohol denat. Is added and dispersed until uniform. Using a propellor stirrer, all materials including the Extruded colloidal oatmeal, are slowly added and stirred until uniform. The product is made to weight using purified water and stirred until uniform.

### Example 37 Skin pH Balancing Toner (Formulation 29)

| | %w/w |
|---|---|
| Aqua | to 100 |
| Alcohol denat. | 7.9 |
| Butylene glycol | 2 |
| Dimethicone copolyol | 1.5 |
| Sodium lactate | 0.6 |
| Glycerin | 0.5 |
| Allantoin | 0.1 |
| Propylene glycol | 0.1 |
| Lactic acid | 0.002 |
| Preservative | q.s |
| Extruded colloidal oatmeal | 1 |

### Method

### Stage 1

Into the water, lactic acid and alcohol denat are separately added and dispersed until uniform. Using a propellor stirrer, all materials including the Extruded colloidal oatmeal, are slowly added and stirred until uniform. The product is made to weight using purified water and stirred until uniform.

### Example 38 Skin pH Balancing Toner (Formulation 30)

| | %w/w |
|---|---|
| Aqua | to 100 |
| Alcohol denat. | 7.9 |
| Butylene glycol | 2 |
| Dimethicone copolyol | 1.5 |
| Sodium lactate | 0.6 |
| Glycerin | 0.5 |
| Allantoin | 0.1 |
| Propylene glycol | 0.1 |
| Lactic acid | 0.002 |
| Preservative | q.s |
| Extruded colloidal oatmeal | 10 |

### Method

### Stage 1

Into the water, lactic acid and alcohol denat are separately added and dispersed until uniform. Using a propellor stirrer, all materials including the Extruded colloidal oatmeal, are slowly added and stirred until uniform. The product is made to weight using purified water and stirred until uniform.

### Example 39 pH Balanced Cleansing Lotion (Formulation 31)

| | %w/w |
|---|---|
| Aqua | to 100 |
| Paraffinum liquidum | 14 |
| Isopropyl palmitate | 7 |
| Glyceryl stearate | 2.5 |
| PEG-100 stearate | 2.5 |
| Butylene glycol | 2 |
| Hydrogenated vegetable glycerides citrate | 2 |
| Polysorbate 60 | 0.5 |
| Sorbitan stearate | 0.5 |
| Persea gratissima | 0.3 |
| Prunus persica | 0.3 |
| Propylene glycol | 0.3 |
| Acrylates/C10-30 alkyl acrylate crosspolymer | 0.12 |
| Potassium hydroxide | 0.05 |
| Tetrasodium EDTA | 0.02 |
| Medicago sativa | 0.0045 |
| Preservative | q.s |
| Extruded colloidal oatmeal | 1 |

### Method

### Stage 1

Into the water, EDTA is added and dispersed. Butylene glycol is then added and dispersed. The aqueous phase is then heated to 70°C.

### Stage 2

The paraffinum liquidum, isopropyl palmitate, glyceryl stearate, PEG-100 stearate, hydrogenated vegetable glycerides citrate, polysorbate 60 and sorbitan stearate are mixed and heated to 70°C to melt the waxes.

### Stage 3

Using a homogeniser, stage 2 is added to stage 1 and this is mixed until emulsified and uniform. The emulsion is then cooled to below 35°C using stirring. The remaining materials, including the Extruded colloidal oatmeal are then added and mixed. The product is then made to weight using purified water and is stirred until uniform.

### Example 40 pH Balanced Cleansing Lotion (Formulation 32)

| | %w/w |
|---|---|
| Aqua | to 100 |
| Paraffinum liquidum | 14 |
| Isopropyl palmitate | 7 |
| Glyceryl stearate | 2.5 |
| PEG-100 stearate | 2.5 |
| Butylene glycol | 2 |
| Hydrogenated vegetable glycerides citrate | 2 |
| Polysorbate 60 | 0.5 |
| Sorbitan stearate | 0.5 |
| Persea gratissima | 0.3 |
| Prunus persica | 0.3 |
| Propylene glycol | 0.3 |
| Acrylates/C10-30 alkyl acrylate crosspolymer | 0.12 |
| Potassium hydroxide | 0.05 |
| Tetrasodium EDTA | 0.02 |
| Medicago sativa | 0.0045 |
| Preservative | q.s |
| Extruded colloidal oatmeal | 10 |

### Method

### Stage 1

Into the water, EDTA is added and dispersed. Butylene glycol is then added and dispersed. The aqueous phase is then heated to 70°C.

### Stage 2

The paraffinum liquidum, isopropyl palmitate, glyceryl stearate, PEG-100 stearate, hydrogenated vegetable glycerides citrate, polysorbate 60 and sorbitan stearate are mixed and heated to 70°C to melt the waxes.

### Stage 3

Using a homogeniser, stage 2 is added to stage 1 and this is mixed until emulsified and uniform. The emulsion is then cooled to below 35°C using stirring. The remaining materials, including the Extruded colloidal oatmeal are then added and mixed. The product is then made to weight using purified water and is stirred until uniform.

### Example 41 Lipstick (Formulation 33)

| | % w/w |
|---|---|
| | |
| **1. RICINUS COMMUNIS** | **20** |
| | |
| Octyldodecanol | 15 |
| Pentaerythrityl tetracaprylate/caprate | 14 |
| Mica | 10 |
| Bis-diglyceryl caprylate/caprate/isostearate/ Stearate/hydroxystearate adipate | 7.5 |
| Paraffin | 5 |
| Cera microcristallina | 5 |
| Propylene glycol | 2 |
| Hydrogenated castor oil | 2 |
| Candelilla cera | 1 |
| Carnauba | 1 |
| Synthetic wax | 1 |
| Butyrospermum parkii | 1 |
| Titanium dioxide | 0.5 |
| Tocopheryl acetate | 0.2 |
| Polyquaternium-37 | 0.2 |
| Pigments and dyes | q.s |
| Extruded colloidal oatmeal | 1 |

### Method

### Stage 1

The Extruded colloidal oatmeal is pre-dispersed in propylene glycol, with stirring.

### Stage 2

The remaining materials are mixed in a vessel and heated to 85°C until melted and uniform. The product is cooled and the Extruded colloidal oatmeal pre-mix is added below 70°C. The product poured into a suitable container and allowed to cool to room temperature to set.

### Example 42 Lipstick (Formulation 34)

| | % w/w |
|---|---|
| | |
| **2. RICINUS COMMUNIS** | **20** |
| | |
| Octyldodecanol | 15 |
| Pentaerythrityl tetracaprylate/caprate | 14 |
| Mica | 10 |
| Bis-diglyceryl caprylate/caprate/isostearate/ Stearate/hydroxystearate adipate | 7.5 |
| Paraffin | 5 |
| Cera microcristallina | 5 |
| Propylene glycol | 2 |
| Hydrogenated castor oil | 2 |
| | |
| **2.1 Candelilla cera** | **1** |
| | |
| Carnauba | 1 |
| Synthetic wax | 1 |
| **Butyrospermum parkii** | **1** |
| Titanium dioxide | 0.5 |
| Tocopheryl acetate | 0.2 |
| Polyquaternium-37 | 0.2 |
| Red colour | q.s |
| Extruded colloidal oatmeal | 10 |

### Method

### Stage 1

The Extruded colloidal oatmeal is pre-dispersed in propylene glycol, with stirring.

### Stage 2

The remaining materials are mixed in a vessel and heated to 85°C until melted and uniform. The product is cooled and the Extruded colloidal oatmeal pre-mix is added below 70°C. The product poured into a suitable container and allowed to cool to room temperature to set.

### Example 43 Hair Conditioner (Formulation 35)

| | %W/W |
|---|---|
| Aqua | to 100 |
| Cetyl alcohol | 3 |
| Cetrimonium chloride | 0.8 |
| Hydroxyethylcellulose | 0.6 |
| Propylene glycol | 0.5 |
| Panthenol | 0.5 |
| Parfum | 0.3 |
| Benzophenone-4 | 0.2 |
| Sodium chloride | 0.1 |
| Wheat amino acids | 0.14 |
| Citric acid | 0.02 |
| Tetrasodium EDTA | 0.02 |
| Extruded colloidal oatmeal | 1 |

### Method

### Stage 1

The EDTA and Hydroxyethylcellulose were added to the water and mixed using a homogeniser to hydrate the polymer. Citric acid, Benzophenone and Cetrimonium chloride were added. This was then heated to 70C.

### Stage 2

Cetyl alcohol was heated to 70C in a separate vessel.

### Stage 3

### The melted Cetyl alcohol was then added to stage 1 using a homogeniser.

### Stage 4

The mixture was then cooled to below 40C using a prop. Stirrer. The remaining materials including the Extruded colloidal oatmeal were then added and the product was made to weight with purified water.

### Example 44 Intensive Conditioner (Formulation 36)

| | %W/W |
|---|---|
| Aqua | to100 |
| Cetearyl alcohol | 4.5 |
| Arachidyl propionate | 2 |
| Dimethicone | 2 |
| Panthenol | 0.75 |
| Stearamidopropyl dimethylamine | 1.5 |
| Hydroxyethylcellulose | 0.5 |
| Amodimethicone | 0.7 |
| Citric acid | 0.5 |
| Cetrimonium chloride | 0.4 |
| PEG-20 stearate | 0.4 |
| Parfum | 0.3 |
| Propylene glycol | 0.3 |
| Benzophenone-4 | 0.2 |
| Sodium chloride | 0.15 |
| Wheat amino acids | 0.15 |
| Polyquaternium-39 | 0.1 |
| Extruded colloidal oatmeal | 1 |

### Method

### Stage 1

The EDTA and HEC were added to the water and mixed using a homogeniser to hydrate the polymer.

### Stage 2

The citric acid and cetrimonium chloride were added and mixed using a prop. Stirrer. The mixture was then heated to 70C.

### Stage 3

In a separate vessel, the waxes, dimethicone and BHT were mixed and heated to 70C until melted and uniform.

### Stage 4

Stage 3 was added to stage 2 and this was mixed until uniform. The mixture was then cooled to below 40C with stirring.

### Stage 5

The remaining materials including the Extruded colloidal oatmeal were then added and the product was made to weight using purified water.

### Example 45 Leave-in Conditioner (Formulation 37)

| | %W/W |
|---|---|
| Aqua | to 100 |
| PEG-40 hydrogenated castor oil | 2 |
| Dipropylene glycol | 1 |
| Phenoxyethanol | 0.8 |
| Parfum | 0.3 |
| Panthenol | 0.4 |
| Propylene glycol | 0.25 |
| Methylparaben | 0.2 |
| Benzophenone-4 | 0.2 |
| Extruded colloidal oatmeal | 1 |

### Method

### Stage 1

### The Polyquaternium-10 was added to the water and hydrated using a prop. stirrer.

### Stage 2

The Methylparaben was pre-dispersed in Dipropylene glycol, gently heated to melt and then added to stage 1.

### Stage 3

The remaining materials including the Extruded colloidal oatmeal were then added and the product was mixed and made to weight with purified water.

### Example 46 Gentle Shampoo (Formulation 38)

| | %W/W |
|---|---|
| Aqua | to100 |
| Sodium laureth sulfate | 8 |
| Cocamidopropyl betaine | 3 |
| Sodium chloride | 1.8 |
| Cocamide DEA | 1.6 |
| PEG-6 cocamide | 1 |
| Parfum | 0.5 |
| Panthenol | 0.4 |
| Propylene glycol | 0.3 |
| Benzophenone-4 | 0.2 |
| Glycerin | 0.2 |
| Phenoxyethanol 1 | 0.1 |
| Extruded colloidal oatmeal | 1 |

### Method

### Stage 1

To the water, EDTA, Sodium chloride, Citric acid and Benzophenone-4 were added. This was followed by the addition of Sodium laureth sulfate, Methyldibromo glutaronitrile, wheat amino acids and the Extruded colloidal oatmeal.

### Stage 2

PEG-6 cocamide and Cocamide DEA were heated gently until liquified. The parfume was added and mixed. This was then added to the product.

### Stage 3

The Cocamidopropyl betaine and remaining materials, including the Extruded colloidal oatmeal were then added and mixed. The product was made to weight using purified water.

### Example 47 Intensive Conditioner (Formulation 39)

| | %W/W |
|---|---|
| Aqua | to 100 |
| Sodium laureth sulfate | 8.5 |
| Cocamidopropyl betaine | 23 |
| Cocamide DEA | 2.2 |
| Panthenol | 1 |
| Sodium chloride | 0.8 |
| Laureth-3 | 0.5 |
| Parfum | 0.3 |
| Propylene glycol | 0.5 |
| Benzophenone-4 | 0.2 |
| Glycerin | 0.5 |
| Phenoxyethanol | 0.5 |
| Extruded colloidal oatmeal | 3 |

### Example 48 Anti-dandruff Shampoo (Formulation 40)

| | %W/W |
|---|---|
| Aqua | to 100 |
| Sodium laureth sulfate | 6 |
| Disodium laureth sulfosuccinate | 4 |
| Laureth-3 | 3 |
| Cocamidopropyl betaine | 2.5 |
| Sodium chloride | 2 |
| Dipropylene glycol | 1 |
| Parfum | 0.5 |
| Piroctone olamine | 0.5 |
| Panthenol | 0.4 |
| Propylene glycol | 0.3 |
| Disodium phosphate | 0.25 |
| Benzophenone-4 | 0.2 |
| Wheat amino acids | 0.15 |
| Extruded colloidal oatmeal | 2 |

### Method

### Stage 1

EDTA, Citric acid and Benzophenone-4 were added and mixed into the water. Sodium laureth sulfate, Disodium laureth sulfosuccinate and Dipropylene glycol were then added.

### Stage 2

Disodium phosphate, wheat amino acids and the Extruded colloidal oatmeal were added and the product was stirred until uniform.

### Stage 3

The Piroctone olamine was dispersed in the parfum and added to the Laureth-3. This mixture was added to the bulk and stirred.

### Stage 4

The remaining materials were then added and the product was made to weight with purified water.

### Example 49 Anti-chlorine Shampoo (Formulation 41)

| | %W/W |
|---|---|
| Aqua | to 100 |
| Sodium laureth sulfate | 8 |
| Cocamidopropyl betaine | 3 |
| Sodium chloride | 1.6 |
| Laureth-3 | 1 |
| Phenoxyethanol | 0.8 |
| Parfum | 0.5 |
| Disodium phosphate | 0.4 |
| Panthenol | 0.4 |
| Propylene glycol | 0.3 |
| Methylparaben | 0.2 |
| Benzophenone-4 | 0.2 |
| Wheat amino acids | 0.14 |
| Propylparaben | 0.1 |
| Sodium thiosulfate | 0.1 |
| Extruded colloidal oatmeal | 1 |

### Stage 1

To the water, the following materials were added and mixed; Benzophenone, Sodium chloride, Sodium phosphate, Disodium phosphate, EDTA.

### Stage 2

Sodium laureth sulfate, phenoxyethanol, Panthenol, Wheat amino acids and the Extruded colloidal oatmeal were then added and stirred.

### Stage 3

The preservatives were pre-mixed in the Laureth-3 and heated slightly to melt the powders. This was added to the product.

### Stage 4

The remaining materials were added and the product was made to weight using purified water.

### Example 50 Hair gel (Formulation 42)

| | %W/W |
|---|---|
| Aqua | to 100 |
| Cyclomethicone | 7 |
| Dimethiconol | 1 |
| Phenoxyethanol | 0.8 |
| Propylene glycol | 0.8 |
| Panthenol | 0.7 |
| Carbomer | 0.7 |
| Aminomethyl propanol | 0.4 |
| Benzophenone-4 | 0.2 |
| Parfum | 0.2 |
| Extruded colloidal oatmeal | 1 |

### Method

### Stage 1

To the water; EDTA and Benzophenone-4 were added using an homogeniser.

### Stage 2

The carbomer was added and hydrated with continued homogenising.

### Stage 3

The Phenoxyethanol, Cyclomethicone, Dimethiconol, Propylene glycol and Panthenol were then added and mixed until homogenous.

### Stage 4

The remaining materials including the Extruded colloidal oatmeal were added and the bulk was homogenised until uniform.

### Stage 5

The product was made to weight using purified water.

### Example 51 Hair Putty (Formulation 43)

| | %W/W |
|---|---|
| Aqua | to 100 |
| Cetearyl alcohol | 11 |
| Lanolin | 7 |
| PVP | 6 |
| Paraffin | 6 |
| PVP/VA copolymer | 6 |
| Carnauba | 3 |
| Petrolatum | 2 |
| Polyquaternium-11 | 2 |
| PEG-20 stearate | 2 |
| Paraffinum liquidum | 1 |
| Propylene glycol | 0.8 |
| Phenoxyethanol | 0.6 |
| Dimethicone | 0.5 |
| Panthenol | 0.4 |
| Cetrimonium chloride | 0.35 |
| Dimethicone propyl PG-betaine | 0.25 |
| Benzophenone-4 | 0.2 |
| Methylparaben | 0.12 |
| Extruded colloidal oatmeal | 3 |

### Method

### Stage 1

To the water, the PVP/VA copolymer, PVP and Benzophenone-4 were added and stirred until homogenous. This was then heated to 70C.

### Stage 2

In a separate vessel, the waxes were mixed and heated to 70C until all materials had melted.

### Stage 3

The hot waxes were then added to stage 1 and mixed using a prop. Stirrer until homogenous. The mixture was then cooled to below 60C.

### Stage 4

The remaining materials, including the Extruded colloidal oatmeal were then added and the product was stirred until uniform.

### Stage 5

The product was made to weight using purified water.

### Example 52 Hydrating Conditioner (Formulation 44)

| | %W/W |
|---|---|
| Aqua | to 100 |
| Cetyl alcohol | 4 |
| Dimethicone | 2 |
| Hydroxyethylcellulose | 0.8 |
| Cetrimonium chloride | 0.8 |
| Panthenol | 0.75 |
| Propylene glycol | 0.6 |
| Parfum | 0.3 |
| Benzophenone-4 | 0.2 |
| Amodimethicone | 0.15 |
| Dimethicone propyl PG-betaine | 0.15 |
| Sodium chloride | 0.15 |
| Extruded colloidal oatmeal | 1 |

### Method

### Stage 1

To the water, EDTA and Hydroxyethylcellulose were added using homogenising to hydrate the polymer.

### Stage 2

The benzophenone-4 and Laureth-3 were then added and the bulk was heated to 70C.

### Stage 3

In a separate vessel, the Cetyl alcohol was heated to 70C until melted.

### Stage 4

Using an homogeniser, the Cetyl alcohol was added to the bulk and mixed until uniform.

### Stage 5

The product was cooled and the remaining materials, including the Extruded colloidal oatmeal were then added and mixed.

### Stage 6

The product was made to weight using purified water.

### Example 53 Spray-On Gel (Formulation 45)

| | %W/W |
|---|---|
| Phase 1 | |
| Aqua | to 100 |
| PVP/VA copolymer | 5 |
| Isopropyl alcohol | 2.5 |
| Propylene glycol | 2.3 |
| Glycerin | 2 |
| Panthenol | 0.4 |
| Benzophenone-4 | 0.2 |
| Extruded colloidal oatmeal | 1 |
| | |

| Phase 2 | |
|---|---|
| PEG-40 hydrogenated castor oil | 1 |
| Parfum | 0.5 |

| Phase 3 | |
|---|---|
| Alcohol denat. | 45 |
| Dimethicone copolyol | 1 |

### Method

### Stage 1

The materials in phase 1 were mixed until uniform using a prop. Stirrer.

### Stage 2

The materials in phase 2 were pre-mixed and added to phase 1.

### Stage 3

The materials in phase 3 were mixed and added to the bulk.

### Stage 4

The product was made to weight using purified water.

### Example 54 Shampoo for Dry Scalps (Formulation 46)

| | %W/W |
|---|---|
| Aqua | to100 |
| Sodium laureth sulfate | 7 |
| Sodium chloride | 2.5 |
| Cocamidopropyl betaine | 2 |
| Laureth-3 | 1 |
| Panthenol | 0.5 |
| Propylene glycol | 0.5 |
| Piroctone olamine | 0.25 |
| Benzophenone-4 | 0.2 |
| Extruded colloidal oatmeal | 1 |

### Method

### Stage 1

To the water, EDTA, Citric acid, Benzophenone-4 and Sodium chloride were added and mixed using a prop. Stirrer until all materials were dissolved and uniform.

### Stage 2

The Sodium laureth sulfate and Piroctone Olamine were then added and stirred until homogenous.

### Stage 3

The remaining materials, including the Extruded colloidal oatmeal were then added and the product was stirred until uniform and homogenous.

### Stage 4

The product was made to weight with purified water.

### Example 55 Deep Cleansing Shampoo (Formulation 47)

| | %W/W |
|---|---|
| Aqua | to 100 |
| Sodium laureth sulfate | 15 |
| Sodium chloride | 2.8 |
| Cocamidopropyl betaine | 1.5 |
| PEG-6 cocamide | 1 |
| Parfum | 0.15 |
| Panthenol | 0.4 |
| Propylene glycol | 0.3 |
| Benzophenone-4 | 0.2 |
| Phenoxyethanol 1 | 0.1 |
| Extruded colloidal oatmeal | 1 |

### Method

### Stage 1

To the water, Citric acid, EDTA and Sodium chloride were added and dissolved.

### Stage 2

The Benzophenone-4, Sodium laureth sulfate, Cocamidopropyl betaine, Panthenol, Methydibromo glutaronitrile, Wheat amino acids and the Extruded colloidal oatmeal were then added and mixed until the product was uniform, using a prop. Stirrer.

### Stage 3

The parfum was pre-dispersed in the PEG-6 cocamide and then added to the bulk.

### Stage 4

The product was made to weight using purified water.

### Example 56 Hydrating Shampoo (Formulation 48)

| | %W/W |
|---|---|
| Aqua | to 100 |
| Sodium laureth sulfate | 8 |
| Cocamidopropyl betaine | 3 |
| Cocamide DEA | 1.7 |
| Panthenol | 1.2 |
| Sodium chloride | 1.5 |
| Laureth-3 | 1.2 |
| Parfum | 0.5 |
| Propylene glycol | 0.5 |
| Polyquaternium-10 | 0.4 |
| Glycerin | 0.2 |
| Extruded colloidal oatmeal | 1 |

### Method

### Stage 1

To the water, the EDTA and Polyquaternium-10 were added and the polymer was hydrated using an homogeniser.

### Stage 2

The Citric acid, Sodium chloride and Benzophenone-4 were added and stirred until uniform.

### Stage 3

The remaining materials, including the Extruded colloidal oatmeal were added individually and the product was mixed using a prop. Stirrer until homogenous.

### Stage 4

The product was made to weight using purified water.

### Example 57 Extra Hold Hair Gel (Formulation 49)

| | %W/W |
|---|---|
| Aqua | to 100 |
| PVP/VA copolymer | 2 |
| Propylene glycol | 1.3 |
| Carbomer | 1 |
| PEG-40 hydrogenated castor oil | 1 |
| Panthenol | 0.6 |
| Sodium hydroxide | 0.3 |
| Parfum | 0.3 |
| Phenoxyethanol | 0.2 |
| Tetrasodium EDTA | 0.1 |
| Mica | 0.1 |
| Cystine hydroxypropyl polysiloxane | 0.1 |
| Extruded colloidal oatmeal | 1 |

### Method

### Stage 1

The EDTA, Methyldibromo glutaronitrile, PVP/VA copolymer and Carbomer were added to the water and mixed using a homogeniser to ensure that the polymers were hydrated.

### Stage 2

With continued homogenising, the Cystine hydroxypropyl polysiloxane was added and mixed into the product.

### Stage 3

The remaining materials, including the Extruded colloidal oatmeal were added individually and mixed using a prop. Stirrer until the product was homogenous.

### Examples of colour cosmetic formulations containing colloidal oatmeal

### Example 58 Pressed Powder (Colour Cosmetics Formulation 1)

| Phase A | |
|---|---|
| | %w/w |
| Talc and methicone 1 | 61.25 |
| Nylon 12 2 | 15.00 |
| Extruded Colloidal Oatmeal | 5.00 |
| Tocopherol/glycerine-vitamin E | 1.40 |
| Iron oxide red 3 | 2.35 |
| Iron oxide yellow 3 | 2.35 |
| Iron oxide black 3 | 0.60 |
| Titanium oxide 4 | 4.00 |
| Preservative/Fragrance | q.s. |

| Phase B | |
|---|---|
| | %w/w |
| BC 2161 Dimethicone and Trimethylsilyloxysilicate 5 | 4.50 |
| BC 2231 Cyclopentasiloxane and Dimethiconol 5 | 3.50 |

### Method

### Stage 1

Combine phase A using a ribbon blender and micropulverise.

### Stage 2

Combine phase B, separately, and heat the mixture to 50°C.

### Stage 3

Spray phase B into the phase A mixture while blending.

### Stage 4

Micropulverise until homogeneous and press at 1200-1500 PSI.

### Example 59 Loose Bronze Powder (Colour Cosmetics Formulation 2)

| Part 1 | |
|---|---|
| Material | %w/w |
| Kobo Mica 127 | 18.15 |
| Mica powder Y 3000 | 15 |
| Polymethyl methacrylate BPA 500 | 5 |
| Talc | to 100 |
| Extruded colloidal Oatmeal | 7 |
| Iron Oxides (C.I. 77492) (And) Isopropyl Titanium Triisostearate (And) Triethoxysilylethyl Polydimethylsiloxyethyl Dimethicone | 0.2 |
| Iron Oxides (C.I. 77491) (And) Isopropyl Titanium Triisostearate (And) Triethoxysilylethyl Polydimethylsiloxyethyl Dimethicone | 0.1 |
| Iron Oxides (C.I. 77499) (And) Triethoxysilylethyl Polydimethylsiloxyethyl Dimethicone (And) Isopropyl Titanium Triisostearate | 0.05 |
| Preservatives | 0.25 |
| | |

| Part 2 | |
|---|---|
| Dimethicone | 4.00 |
| dl-alpha Tocopherol Acetate | 1.00 |
| | |

| Part 3 Pearls | |
|---|---|
| Mixed colours pearls Mica (And) Iron Oxides (C.I. 77491) | 30 |
| Mixed colours pearls Mica (And) Titanium Dioxide (And) Iron Oxides (C.I. 77491) | 3.5 |
| Mixed colours pearls Titanium Dioxide (And) Mica (And) Iron Oxides (C.I. 77491) | 2.5 |

### Method

### Stage 1

Weigh and add Part 1 raw materials to the Waring Blender under a fume hood. Mix for 2 -3 minutes.

### Stage 2

Pre-mix Part 2.

### Stage 3

Add Part 2 slowly to Part 1 under a fume hood. Mix for 2 minutes.

### Stage 4

Add the Pearls and mix for 1-2 minutes.

### Example 60 Creme - Powder Blush (Colour Cosmetics Formulation 3)

| Part 1 | %w/w |
|---|---|
| **SERICITE GMS-4C** Mica | 17 |
| **RBTD-I2** Titanium Dioxide (And) Isopropyl Titanium Triisostearate | 10 |
| Extruded Colloidal Oatmeal | 3 |
| **SPC/MST-547-I2** Polymethylsilsesquioxane (And) Copolymer Ethylene/Methacrylate (And) Isopropyl Titanium Triisostearate | 6 |
| **BYO-I2** Iron Oxides (C.I. 77492) (And) Isopropyl Titanium Triisostearate | 0.33 |
| **BRO-I2** Iron Oxides (C.I. 77491) (And) Isopropyl Titanium Triisostearate | 0.33 |
| **BBO-I2** Iron Oxides (C.I. 77499) (And) Isopropyl Titanium Triisostearate | 0.1 |
| Preservatives | 0.2 |
| | |
| Part 2 | |
| Wickenol 155 Ethylhexyl Palmitate | 32.55 |
| Squalane NF Squalane | 12.00 |
| Microcrystalline Wax SP-89 Microcrystalline Wax | 6.00 |
| Lameform TGI Polyglycerol-3 Diisostearate | 5.50 |
| Mineral Oil Carnation Mineral Oil | 3.00 |
| Softisan® 100 Hydrogenated Coco-Glycerides | 2.00 |
| Carnauba Wax SP 63 Copernicia Cerifera (Carnauba) Wax | 2.00 |

### Method

### Stage 1

Blend Part 1 and pass through a micronizer until the color is fully dispersed.

### Stage 2

Heat Part 2 with stirring to 91-93°C. Maintain temperature for 30 minutes.

### Stage 3

Add Part 1 to Part 2 and mix until homogeneous. Stir and cool to 88°C. Add Part 3.

### Stage 4

Continue to mix until uniform while maintaining temperature. Fill at 85°C.

### Example 61 Non-transfer Lipstick (Colour Cosmetics Formulation 4)

| Part 1 | %w/w |
|---|---|
| **KOBOGUARD® HRPC** Hydrogenated Polycyclopentadiene (And) Polyethylene (And) Copernicia Cerifera (Carnauba) Wax (And) Tocopherol | 20.00 |
| Extruded colloidal oatmeal | 5 |
| **INBP75ER** Iron Oxides (C.I. 77491) (And) Isononyl Isononanoate (And) Isopropyl Myristate (And) Stearalkonium Hectorite (And) Polyhydroxystearic Acid (And) Isopropyl Titanium Triisostearate (And) Propylene Carbonate | 7.25 |
| Candelilla Wax SP 75 Euphorbia Cerifera (Candelilla) Wax | 7.00 |
| **SERICITE GMS-4C** Mica | 5.00 |
| Ozokerite Wax White SP 1020P Ozokerite | 4.50. |
| Microcrystalline Wax SP-89 Microcrystalline Wax | 2.00 |
| | |

| Part 2 | |
|---|---|
| Permethyl 99AD Isododecane | 49.25 |

### Method

*Use explosion-proof mixers and equipment during batching process*

### Stage 1

Combine Part 1 and heat to 90°C. Mix well under propeller until color is fully dispersed.

### Stage 2

Cool to 80°C and add Part 2.

### Stage 3

Pour into molds.

### References:

Asp N.-G, Björck I. Extrusion cooking and food safety. In book Extrusion cooking, published. C. Mercier, P. Linko and J.M. Harper, American Association of cereal chemists Inc., USA 1998, 413
Cheftel, J. C., Extrusion cooking and food safety. In book Extrusion cooking, published. C. Mercier, P. Linko and J.M. Harper, American Association of cereal chemists Inc., USA 1998, 450-456
Ilo, S., Schoenlenger R. and Berghofe E., Role of lipids in the extrusion cooking processes, Grasas y Aceitas vol. 51 Fasc. 1-2 (2000), 97 - 110
Kurtz, E. and Wallo, W., Colloidal oatmeal: History, chemistry and clinical properties, Journal of Drugs in Dermatology 6 (2) (2007), 167-170.
Maunsell C., Beerling J. and Fielder D. Oats as a cosmetic ingredient: History and current applications, Personal Care, 2011 March
Pillai, R., Redmond, M., Röding, J. Significant New Findings in the Non-Invasive Cosmetic Treatment of Skin Wrinkles with Beta-Glucan, IFSCC Magazine, 8, (1), (2005)
Pomeranz Y., Industrial uses of oats, in book The Oat Crop, published. Welch R. W. Chapman & Hall, UK 1995 1st edition, 490
United States Pharmacopeial Convention USP 32 -NF27, page 2024 describes the raw materials required for oatmeal, packaging and storage, identification, viscosity (including the method of measurement and apparatus), microbial enumeration tests and tests for specified microorganisms, loss on drying, particle size, total ash, fat content, and nitrogen content.
The United States Pharmacopeia (USP) is the official pharmacopeia of the United States, published dually with the National Formulary as the USP-NF. The United States Pharmacopeial Convention (usually also called the USP) is the non-profit organization that owns the trademark and copyright to the USP-NF and publishes it every year. Prescription and over-the-counter medicines and other health care products sold in the United States are required to follow the standards in the USP-NF. USP also sets standards for food ingredients and dietary supplements.
Food and Drug Administration [FDA] Monograph Vol. 69, No. 160 2004 describes Colloidal Oatmeal as classified as a skin protectant by the FDA according to 21 CFR Part 310 and 347 in Skin Protectant Drug Product Over-the-Counter Human Use: Final Monograph. This is contained in the Federal Register Vol. 69, No. 160, Thursday, August 19,2004
Wang S. S. Gelatinisation of Melting of Starch and Tribochemistry in Extrusion, Starch/Stärke 45 (1993) 11 388-390.
Gutkoski L.C. and El-Dash A.A Effect of extrusion process variables on physical and chemical properties of extruded oat products, Plant Foods for Human Nutrition 54: 315 - 325, 1999.

This invention relates to novel cereal products, especially natural oat flour products of colloidal nature. The invention also relates production and uses of colloidal oat flour products. Further the invention relates to cosmetic formulations containing colloidal oat flour products.

## Claims

1. A colloidal oat flour product produced by extrusion at a temperature in the range of 110 °C - 120 °C and a moisture content of below 17 w % and fine grinding, the oat flour product having a solubility corresponding to a value of over 0.4 w % dissolved solids evaluated in 10 w % water suspension according to the Brix method, and having a microbial count less than 5000 cfu/g.

2. The product of claim 1 that is natural.

3. The product of claim 1 having a microbial count less than 1000 cfu/g.

4. The product of claim 1 having a microbial count less than 100 cfu/g.

5. The product of any of preceding claims having a viscosity of at least 0.150 Pas measure at 20°C from a water suspension containing 10 w % of dry matter at a shear rate in the range of 20 to 40 l/s.

6. The product of any of preceding claims having improved oil absorption properties compared to non-extruded flour product.

7. A method for producing a colloidal oat flour product according to any of claims 1 to 6 comprising the steps of extruding the oat flour and fine grinding, wherein the conditions in extrusion chamber are controlled by
a) maintaining the moisture content of the feed below 17 w %, among others by maintaining a temperature in the range of 110 °C - 120 °C;
and
b) adding oil(s) to the feed at 1 to 6 w %;
and/or
c) lowering pressure of the extrusion chamber by 20 % compared to conventional pressures that are in the range 50-150 bar.

8. A method of claim 7 wherein the conditions in extrusion chamber are controlled by
a) maintaining the moisture content of the feed below 15 w %;
and
b) adding oil(s) to the feed at 2 to 4 w %;
and/or
c) lowering pressure of the extrusion chamber by a total of 40 w % compared to conventional pressures.

9. A method of claim 7 or 8, wherein the conditions in extrusion chamber are controlled by
a) maintaining the moisture content of the feed below 13 w %;
and
b) adding oil(s) to the feed to about 3 w %;
and/or
c) lowering pressure of the extrusion chamber by a total of 60 w % compared to conventional pressures.

10. The method of any of claims 7 to 9 wherein the pressure is lowered by the use of a reverse screw.

11. Use of the product of any of claims 1 to 6 or product obtained according to the method of any of claim 7 to 10 in a cosmetic formulation.

12. Use of the product of any of claims 1 to 6 or product obtained according to the method of any of claim 7 to 10 in a food product.

13. Use of the product of any of claims 1 to 6 or product obtained according to the method of any of claim 7 to 10 as a coating barrier in protective products.

14. A cosmetic product containing a product of any of claims 1 to 6 or product obtained according to the method of any of claim 7 to 10.

15. A cosmetic product of claim 14 being a bath powder containing 10 to 60 w % of a product of any of claims 1 to 6 or product obtained according to the method of any of claim 7 to 10.

16. A cosmetic product of claim 14, being baby balm, face mask or body lotion containing 1 to 10 w % of a product of any of claims 1 to 6 or product obtained according to the method of any of claim 7 to 10.

## Patentansprüche

1. Kolloidales Hafermehlprodukt, hergestellt mittels Extrusion bei einer Temperatur in einem Bereich von 110°C - 120°C und einem Feuchtigkeitsgehalt von unter 17 Gew.-% und feinem Vermahlen, wobei das Hafermehlprodukt eine Löslichkeit aufweist, die einem Wert von über 0,4 Gew.-% gelöster Feststoffe entspricht, ausgewertet in 10 Gew.-% wässriger Suspension gemäß dem Brix-Verfahren, und mit einer Mikroben-Anzahl von weniger als 5000 cfu/g.

2. Produkt nach Anspruch 1, das natürlich ist.

3. Produkt nach Anspruch 1 mit einer Mikroben-Anzahl von weniger als 1000 cfu/g.

4. Produkt nach Anspruch 1 mit einer Mikroben-Anzahl von weniger als 100 cfu/g.

5. Produkt nach einem der vorangehenden Ansprüche mit einer Viskosität von mindestens 0,150 Pas messe bei 20°C aus einer wässrigen Suspension enthaltend 10 Gew.-% trockener Substanz bei einer Schergeschwindigkeit im Bereich von 20 bis 40 1/s.

6. Produkt nach einem der vorangehenden Ansprüche mit im Vergleich zu einem nicht-extrudierten Mehlprodukt verbesserten Ölabsorptionseigenschaften.

7. Verfahren zur Herstellung eines kolloidalen Hafermehlprodukts nach einem der Ansprüche 1 bis 6, umfassend die Schritte Extrudieren des Hafermehls und feines Vermahlen, wobei die Bedingungen in der Extrusionskammer gesteuert werden durch
a) Aufrechterhalten des Feuchtigkeitsgehalts der Beschickung unter 17 Gew.-%, unter anderem durch Aufrechterhalten einer Temperatur im Bereich von 110°C - 120°C; und
b) Zufügen von Öl(en) zu der Beschickung mit 1 bis 6 Gew.-%; und/oder
c) Senken eines Drucks der Extrusionskammer um 20% im Vergleich zu herkömmlichen Drücken, welche im Bereich von 50-150 bar liegen.

8. Verfahren nach Anspruch 7, wobei die Bedingungen in der Extrusionskammer gesteuert werden durch
a) Aufrechterhalten des Feuchtigkeitsgehalts der Beschickung unter 15 Gew.-%; und
b) Zufügen von Öl(en) zu der Beschickung mit 2 bis 4 Gew.-%; und/oder
c) Senken eines Drucks der Extrusionskammer um insgesamt 40% im Vergleich zu herkömmlichen Drücken.

9. Verfahren nach Anspruch 7 oder 8, wobei die Bedingungen in der Extrusionskammer gesteuert werden durch
a) Aufrechterhalten des Feuchtigkeitsgehalts der Beschickung unter 13 Gew.-%; und
b) Zufügen von Öl(en) zu der Beschickung bis etwa 3 Gew.-%; und/oder
c) Senken eines Drucks der Extrusionskammer um insgesamt 60% im Vergleich zu herkömmlichen Drücken.

10. Verfahren nach einem der Ansprüche 7 bis 9, wobei der Druck durch die Verwendung einer entgegengesetzten Schraube gesenkt wird.

11. Verwendung des Produkts nach einem der Ansprüche 1 bis 6 oder des nach dem Verfahren nach einem der Ansprüche 7 bis 10 erhaltenen Produkts in einer kosmetischen Formulierung.

12. Verwendung des Produkts nach einem der Ansprüche 1 bis 6 oder des nach dem Verfahren nach einem der Ansprüche 7 bis 10 erhaltenen Produkts in einem Nahrungsmittelprodukt.

13. Verwendung des Produkts nach einem der Ansprüche 1 bis 6 oder des nach dem Verfahren nach einem der Ansprüche 7 bis 10 erhaltenen Produkts als Beschichtungsbarriere in Schutzprodukten.

14. Kosmetisches Produkt enthaltend ein Produkt nach einem der Ansprüche 1 bis 6 oder ein nach dem Verfahren nach einem der Ansprüche 7 bis 10 erhaltenes Produkt.

15. Kosmetisches Produkt nach Anspruch 14, welches ein Badepulver ist, das 10 bis 60 Gew.-% eines Produktes nach einem der Ansprüche 1 bis 6 oder eines nach dem Verfahren nach einem der Ansprüche 7 bis 10 erhaltenen Produkts enthält.

16. Kosmetisches Produkt nach Anspruch 14, welches ein Babybalsam, eine Gesichtsmaske oder eine Körperlotion ist, welches 1 bis 10 Gew.-% eines Produktes nach einem der Ansprüche 1 bis 6 oder eines nach dem Verfahren nach einem der Ansprüche 7 bis 10 erhaltenen Produkts enthält.

## Revendications

1. Produit de farine d'avoine colloïdale produit par extrusion à une température dans la plage de 110°C à 120°C et une teneur en humidité en dessous de 17% en poids et un broyage fin, le produit de farine d'avoine ayant une solubilité correspondant à une valeur de plus de 0,4% en poids des solides dissous évaluée dans 10% en poids de suspension aqueuse selon la méthode Brix, et ayant un dénombrement microbien inférieur à 5000 ufc/g.

2. Produit selon la revendication 1 qui est naturel.

3. Produit selon la revendication 1 ayant un dénombrement microbien inférieur à 1000 ufc/g.

4. Produit selon la revendication 1 ayant un dénombrement microbien inférieur à 100 ufc/g.

5. Produit selon l'une quelconque des revendications précédentes ayant une viscosité d'au moins 0,150 Pa.s mesurée à 20°C à partir d'une suspension aqueuse contenant 10% en poids de matière sèche à une vitesse de cisaillement dans la plage de 20 à 40 l/s.

6. Produit selon l'une quelconque des revendications précédentes ayant des propriétés d'absorption d'huile améliorées par rapport au produit de farine non extrudé.

7. Procédé de production d'un produit de farine d'avoine colloïdale selon l'une quelconque des revendications 1 à 6 comprenant les étapes d'extrusion de la farine d'avoine et de broyage fin, dans lequel les conditions dans la chambre d'extrusion sont commandées par
a) maintenir la teneur en humidité de l'alimentation en dessous de 17% en poids, parmi d'autres en maintenant une température dans la plage de 110°C à 120°C ;
et
b) ajouter de l'huile(s) à l'alimentation à 1 à 6% en poids ;
et/ou
c) abaisser la pression de la chambre d'extrusion de 20% par rapport aux pressions conventionnelles qui sont dans la plage de 50 à 150 bar.

8. Procédé selon la revendication 7 dans lequel les conditions dans la chambre d'extrusion sont commandées par
a) maintenir la teneur en humidité de l'alimentation en dessous de 15% en poids ;
et
b) ajouter de l'huile(s) à l'alimentation à 2 à 4% en poids ;
et/ou
c) abaisser la pression de la chambre d'extrusion d'un total de 40% en poids par rapport aux pressions conventionnelles.

9. Procédé selon la revendication 7 ou 8, dans lequel les conditions dans la chambre d'extrusion sont commandées par
a) maintenir la teneur en humidité de l'alimentation en dessous de 13% en poids ;
et
b) ajouter de l'huile(s) à l'alimentation à environ 3% en poids ;
et/ou
c) abaisser la pression de la chambre d'extrusion d'un total de 60% en poids par rapport aux pressions conventionnelles.

10. Procédé selon l'une quelconque des revendications 7 à 9 dans lequel la pression est abaissée par l'utilisation d'une vis inverse.

11. Utilisation du produit selon l'une quelconque des revendications 1 à 6 ou du produit obtenu d'après le procédé selon l'une quelconque des revendications 7 à 10 dans une formulation cosmétique.

12. Utilisation du produit selon l'une quelconque des revendications 1 à 6 ou du produit obtenu d'après le procédé selon l'une quelconque des revendications 7 à 10 dans un produit alimentaire.

13. Utilisation du produit selon l'une quelconque des revendications 1 à 6 ou du produit obtenu d'après le procédé selon l'une quelconque des revendications 7 à 10 comme une barrière de revêtement dans des produits de protection.

14. Produit cosmétique contenant un produit selon l'une quelconque des revendications 1 à 6 ou un produit obtenu d'après le procédé selon l'une quelconque des revendications 7 à 10.

15. Produit cosmétique selon la revendication 14 étant une poudre de bain contenant 10 à 60% en poids d'un produit selon l'une quelconque des revendications 1 à 6 ou un produit obtenu d'après le procédé selon l'une quelconque des revendications 7 à 10.

16. Produit cosmétique selon la revendication 14, étant un baume pour bébé, un masque facial ou une lotion corporelle contenant 1 à 10% en poids d'un produit selon l'une quelconque des revendications 1 à 6 ou un produit obtenu d'après le procédé selon l'une quelconque des revendications 7 à 10.
